# EUROPEAN PATENT APPLICATION

(11) **EP 4 467 567 A1**
(43) Date of publication of application: **27.11.2024**
(21) Application number: 22921389.7
(22) Date of filing: 15.06.2022
(51) Int. Cl.: C07K 16/28, C12N 15/13, C12N 5/00, A61K 39/395, A61P 35/00, G01N 33/574

(54) **ANTI-CD70 NANOANTIBODY AND USE THEREOF**

(30) Priority: 19.01.2022 CN 202210059899
(71) Applicant: Hrain Biotechnology Co., Ltd., Shanghai 201210 (CN)
(72) Inventor: HUANG, Hui, Shanghai 201210 (CN); PENG, Tao, Shanghai 201210 (CN); HU, Hongming, Shanghai 201210 (CN); LIU, Xiang, Shanghai 201210 (CN); CHEN, Yan, Shanghai 201210 (CN)
(74) Representative: Louis Pöhlau Lohrentz
(86) International application number: PCT/CN2022/098843
(87) International publication number: WO 2023/137958

(57) **Abstract**

The present invention relates to an anti-CD70 nanoantibody and a use thereof. The present invention provides a CD70 binding molecule, containing the anti-CD70 nanoantibody or an antigen binding fragment thereof. Complementarity-determining regions (CDRs) of the anti-CD70 nanoantibody comprise CDR1, CDR2 and CDR3. The present invention also provides a chimeric antigen receptors containing the CD70 binding molecule and an expression cell thereof. The antibody and the cell of the present disclosure have good safety and therapeutic effects targeting CD70.

## Description

### Cross References to Related Applications

This disclosure claims the priority of the Chinese patent application with the application number "CN 202210059899.9" titled "Anti-CD70 Nanobody and Its Use" filed with the Chinese Patent Office on January 19, 2022, the entire contents of which are incorporated by reference in this disclosure middle.

### FIELD OF THE INVENTION

The present description relates to the field of bioimmunotherapy technology, and specifically to anti-CD70 nanoantibody and its application.

### BACKGROUND OF THE INVENTION

CD70 is a member of the tumor necrosis factor (TNF) superfamily. In normal tissues, CD70 is a type II transmembrane glycoprotein, which is only transiently expressed in activated T cells, B cells and mature dendritic cells. CD27 is its receptor. In pathological state, it has been found that CD70 is highly expressed in a variety of tumor cells. Currently, immunotherapeutic drugs targeting CD70 have been applied in preclinical studies.

Studies have shown that the expression of CD70 is abnormally elevated in a variety of malignant tumors, including renal cell carcinoma, acute myeloid leukemia, non-Hodgkin's lymphoma, multiple myeloma, mantle cell lymphoma, diffuse large cell lymphoma, Follicular lymphoma, pancreatic cancer, Breast cancer, glioblastoma and other tumors, and that it is a promising target for tumor immunotherapy.

Chimeric Antigen Receptor-T cells (CAR-T) are a new type of immunotherapy targeting specific antigens on the surface of tumor cells. Now, many researchers are studying CAR-T cells to treat solid tumors.

As an important part of CAR, antibodies have a decisive impact on CAR-T's specific and efficient killing effect and reduction of toxicity. Therefore, antibodies that can bind to target antigens with high specificity and low immunogenicity are the focus of developing CAR-T products. Alpacas can produce high-specific and high-affinity VHH antibodies. At the same time, due to the characteristics of VHH antibodies themselves, they can recognize epitopes that traditional scFv antibodies cannot recognize. In addition, the alpaca VHH gene is highly homologous to the human VH gene, and so it has a relatively low level of immunogenicity, which is also conducive to humanization transformation. There is no report on CAR-T cells containing anti-CD70 nanoantibody in the field.

### SUMMARY OF THE INVENTION

The present description provides a CD70 binding molecule, comprising an anti-CD70 nanoantibody or an antigen-binding fragment thereof, wherein the complementarity determining region (CDRs) of the anti-CD70 nanoantibody comprise CDR1, CDR2 and CDR3, wherein CDR1 comprises the sequence shown by any one of SEQ ID NO: 1-7, CDR2 comprises the sequence shown by any one of SEQ ID NO: 8-13, and CDR3 comprises the sequence shown by any one of SEQ ID NO: 14-20.

In one or more embodiments, the heavy chain variable region sequence of the anti-CD70 nanoantibody is as shown by any one of SEQ ID NO: 21-28.

In one or more embodiments, the FR1 of the anti-CD70 nanoantibody can be selected from FR1 of the VHH shown by any one of SEQ ID NO: 21-28, FR2 can be selected from FR2 of the VHH shown by any one of SEQ ID NO: 21-28, FR3 can be selected from FR3 of the VHH shown by any one of SEQ ID NO: 21-28, FR4 can be selected from FR4 of the VHH shown by any one of SEQ ID NO: 21-28,

In one or more embodiments, the CD70 binding molecules are monovalent or multivalent nanoantibodies or single-domain antibodies, or multispecific nanoantibodies or single-domain antibodies, comprising one, two or more anti-CD70 nanoantibodies or antigen-binding fragment thereof.

In one or more embodiments, the multivalent binding molecule or multispecific binding molecule links to multiple anti-CD70 nanoantibodies or antigen-binding fragments thereof via linkers. The linker consists of 1-15 amino acids selected from G and S.

In one or more embodiments, the nanoantibody is a camelid heavy chain antibody or a shark heavy chain antibody.

In one or more embodiments, the nanoantibody further comprises a heavy chain constant region.

In one or more embodiments, the heavy chain constant region is a constant region of camelid heavy chain antibody, comprising CH2 and CH3. In one or more embodiments, the CH2 and CH3 are CH2 and CH3 of human IgG Fc, such as CH2 and CH3 of IgG1. Preferably, the heavy chain constant region is shown by SEQ ID NO: 37.

In one or more embodiments, the heavy chain constant region is a constant region of the shark heavy chain antibody, comprising CH1, CH2, CH3, CH4, and CH5.

In one or more embodiments, the CD70 binding molecule described in any embodiment of the present description is a chimeric antibody or a fully human antibody; preferably, a fully human antibody.

The present description provides a chimeric antigen receptor, comprising an optional signal peptide sequence, the CD70 binding molecule described in any embodiment herein, a hinge region, a transmembrane region, and an intracellular region.

In one or more embodiments, the intracellular domain comprises an intracellular costimulatory domain and/or an intracellular signaling domain.

In one or more embodiments, the chimeric antigen receptor comprises a signal peptide, CD70 binding molecule described in any embodiment herein, a hinge region, a transmembrane region, an intracellular costimulatory domain, and an intracellular signaling domain in sequence from N-terminal to C-terminal.

The present description also provides a nucleic acid molecule comprising a sequence selected from any of the group consisting of:
(1) the coding sequence of a CD70 binding molecule or chimeric antigen receptor described in any one embodiment herein;
(2) a complementary sequence of (1);
(3) a 5-50 bp fragment of any sequence of (1) or (2).

In one or more embodiments, the fragment is a primer.

The present description also provides a nucleic acid construct comprising the nucleic acid molecule as described herein.

In one or more embodiments, the nucleic acid construct is a cloning vector, expression vector, or integration vector.

The present description also provides a host cell selected from:
(1) expressing and/or secreting the CD70 binding molecule or chimeric antigen receptor described in any one embodiment herein;
(2) comprising a a nucleic acid molecule described herein; and/or
(3) comprising a nucleic acid construct described herein.

In one or more embodiments, the host cell is an immune effector cell, preferably a T cell.

The present description also provides a method for producing a CD70 binding molecule according to any one of the embodiments herein, comprising:
culturing the host cells described herein under conditions suitable for producing the CD70 binding molecule (such as nanoantibodies or antigen binding fragments thereof, monovalent or multivalent nanoantibodies or single domain antibodies, or multispecific nanoantibodies or single domain antibodies), and
optionally purifying the CD70 binding molecule from culture.

The present description also provides a pharmaceutical composition, comprising the CD70 binding molecule, nucleic acid molecule, nucleic acid construct or host cell according to any embodiment herein, and a pharmaceutically acceptable excipient.

In one or more embodiments, the pharmaceutical composition is used to treat a disease or condition associated with CD70 expression.

The present description also provides the use of the CD70 binding molecule, chimeric antigen receptor, nucleic acid molecule, nucleic acid construct or host cell described in any embodiment herein in the preparation of activated immune cells (such as T cells).

The present description also provides the use of the CD70 binding molecule, chimeric antigen receptor, nucleic acid molecule, nucleic acid construct or host cell described in any embodiment herein in the preparation of a medicament for preventing or treating a disease or condition related to CD70 expression.In one or more embodiments, the disease or condition is selected from one or more of the following: renal cell carcinoma, acute myelogenous leukemia, non-Hodgkin's lymphoma, multiple myeloma, mantle cell lymphoma, diffuse large cell lymphoma, follicular lymphoma, pancreatic cancer, breast cancer, glioblastoma.

The description also provides a method for treating or preventing a disease or condition associated with CD70 expression, comprising administrating a patient in need thereof an effective amount of a CD70 binding molecule or the host cells according to any embodiment of the description, or a pharmaceutical compisiton of any embodiment.

The present description also provides a kit for detecting CD70, for use in evaluating the therapeutic effect of a medicament or diagnosing cancer. The kit comprises a CD70 binding molecule, nucleic acid molecule, nucleic acid construct or host cell according to any embodiment of the present description.

In one or more embodiments, the kit further comprises reagents for detecting the binding of CD70 to the CD70 binding molecule. For example, the bound reagent is detected by the enzyme-linked immunosorbent assay.

In one or more embodiments, the detection reagent for binding is a detectable marker, such as biotin, that can be linked to a CD70 binding molecule. The detectable marker is connected to the CD70 binding molecule or present in the kit separately.

The present description also provides a non diagnostic method for detecting the presence of CD70 in a sample. The method comprises: incubating a CD70 binding molecule according to any embodiment herein with the sample, and detecting the binding of CD70 to a CD70 binding molecule, thereby determining the presence of CD70 in the sample. The detection is an enzyme-linked immunosorbent assay.

The present description also provides the use of the CD70 binding molecule according to any embodiment herein in the preparation of a kit for detecting CD70 in a sample, evaluating the therapeutic effect of a medicament or diagnosing a cancer.

The present description has the following beneficial effects:
The present description provides a new mini-antibody that specifically recognizes CD70 and CAR-modified cells containing the antibody, which has a favorable safety profile and therapeutic efficacy in targeting CD70, providing a new therapeutic or ameliorative pathway for diseases associated with CD70 expression.

### Description of drawings

In order to illustrate the technical solutions of the embodiments of the present description more clearly, the accompanying drawings used in the embodiments will be briefly introduced below. It should be understood that the following drawings only show some embodiments of the present description, and should not be regarded as a limitation on the scope, and those skilled in the art can also obtain other related drawings based on these drawings without creative work.
Fig. 1 is the SDS-PAGE electrophoresis result of the recombinant human CD70-huFc recombinant protein in Example 1.
Fig. 2 is the experimental result of the determination of the binding curve between recombinant human CD70-huFc protein and recombinant human CD27 protein in Example 1.
Figure 3 is the result of DNA electrophoresis after PCR amplification of alpaca VH-CH2 gene in Example 2.
Figure 4 is the result of DNA electrophoresis after PCR amplification of alpaca VHH gene in Example 2.
Fig. 5 is the DNA electrophoresis result of the VHH/pcomb3X ligation efficiency detection experiment in Example 2.
Fig. 6 is the SDS-PAGE electrophoresis result of the recombinant VHH-huFc antibody protein in Example 3.
Fig. 7 is the experimental result of the determination of the binding curve of the recombinant VHH-huFc antibody/CD70-huFc recombinant protein in Example 4.
Fig. 8 is the experimental result of the affinity determination of the recombinant VHH-huFc antibody protein in Example 5.
Fig. 9 is the result of the recombinant VHH-huFc blocking CD27/CD70 binding experiment in Example 6.
Fig. 10 is a schematic diagram of the CAR structure in Example 7.
Fig. 11 is the experimental results of the infection efficiency of CD70 CAR-T cells with different clones in Example 8.
Fig.12 is the experimental results of the CD107a activation experiment of different cloned CD70 CAR-T cells in Example 9.
Fig. 13 is the experimental results of IFN-γ release when CD70 CAR-T cells of different clones were co-incubated with target cells in Example 9.
Fig. 14 is the experimental results of IL-2 release when CD70 CAR-T cells of different clones were co-incubated with target cells in Example 9. REST is the NT cell control group.
Fig.15 is the experimental results of the killing experiments of different cloned CD70 CAR-T cells in Example 9.
Fig.16 is the experimental results of the infection efficiency of CD70 CAR-T cells with different clones in Example 10.
Fig.17 is the experimental results of the CD107a activation experiment of different cloned CD70 CAR-T cells in Example 11.
Fig.18 is the experimental results of the killing experiments of different cloned CD70 CAR-T cells in Example 9.
Figure 19 is the tumor volume monitoring results of the in vivo drug efficacy test of CD70 CAR-T cells with different clones in Example 11.
Figure 20 is the T cell survival results of the in vivo drug efficacy test of CD70 CAR-T cells with different clones in Example 11.

### DETAILED DESCRIPTION

The inventors, after extensive and in-depth research and after a large number of screenings, have discovered a class of anti-CD70 nanoantibodies and antigen-binding fragments thereof, which are capable of specifically recognizing CD70, binding to CD70 with high affinity, and are able to block the binding of CD70 to CD27, and have good functional activity.

Specifically, the present description immunizes alpaca with CD70 protein to obtain a high-quality immune single domain antibody gene library. The antibody gene library was then screened by phage display technology to obtain the CD70-specific single domain antibody gene. Then the gene was transfered to mammalian cells, and an antibody strain with high specificity and high expression in mammalian cells was obtained. Nanoantibodies with high affinity, high specificity and high functional activity were then identified by ELISA, molecular interactions analysis and blocking assay. The antibody or antigen-binding fragment thereof has good safety and targeting, and can specifically bind to the extracellular domain of human CD70.

The present description also provides a chimeric antigen receptor (CAR) comprising the nanoantibody. The vector containing the coding sequence of the CAR is used to infect immune cells, and immune effector cells with significant killing ability to tumor cells overexpressing CD70 can be obtained, and the immune effector cells can be applied to treat or improve CD70 expression-related diseases, thereby laying the foundation for the treatment of CD70-positive tumors.

### Antibody

Herein, a "CD70 binding molecule" is a protein that specifically binds CD70, including, but not limited to, antibodies, heavy chain antibodies, nanoantibodies, or antigen-binding fragments thereof.

Term "antibody" described herein comprises monoclonal antibodies (including full-length antibodies with immunoglobulin Fc region), antibody compositions with polyepitopic specificity, multi-specific antibodies (e.g., bispecific antibodies), diabodies and single chain molecules, and antibody fragments, especially antigen binding fragments, (e.g., Fab, F(ab')2, and FV). Herein, "antibody" and "immunoglobulin" are used interchangeably.

The traditional "antibody" contains the basic 4-chain antibody unit and it is a heterotetrameric glycoprotein composed of two identical light chains (L) and two identical heavy chains (H). Each heavy chain has a variable domain (VH) at the N-terminus, followed by three constant domains (for each α and γ chain, CH1, CH2, and CH3) and four constant domains (for µ and ε isoforms, CH1, CH2, CH3, and CH4) and the hinge region (Hinge) between the CH1 domain and the CH2 domain. Each light chain has a variable domain (VL) at the N-terminus, followed by a constant domain (CL) at the other end. The paired VH and VL together form an antigen binding site. For the structures and properties of different classes of antibodies, see, for example, Basic and Clinical Immunology, 8th Edition, Daniel P. Sties, Abba I. Terr, and Tristram G. Parsolw, ed, Appleton & Lange, Norwalk, CT, 1994, page 71, and Chapter 6. The L chain from any vertebrate species can be assigned to one of two clearly distinct types, called κ and λ, based on the amino acid sequences of their constant domains. The γ and α classes are further divided into subclasses on the basis of relatively minor differences in the CH sequence and function, e.g., humans express the following subclasses: IgGl, IgG2A, IgG2B, IgG3, IgG4, IgA1 and IgA2.

"Heavy chain antibody" described herein is an antibody derived from camelidae or sharks. Compared with the above 4-chain antibody, the heavy chain antibody lacks light chain and heavy chain constant region 1 (CH1), and only contains two heavy chains composed of variable region (VHH) and other constant regions, wherein the variable region is linked to the constant region through a hinge region like structure. Each heavy chain of camelid heavy chain antibody comprises one variable region (VHH) and two constant regions (CH2 and CH3), and each heavy chain of shark heavy chain antibody comprises one variable region and five constant regions (CH1-CH5). Antigen binding fragments of heavy chain antibodies comprise VHH and single chain heavy chain antibodies. Heavy chain antibodies may have CH2 and CH3 of human IgG Fc by fusing with the constant region of human IgG Fc.

As described herein, the terms "single domain antibody", "anti-CD70 single domain antibody", "heavy chain variable region domain of heavy chain antibody" and "VHH" can be used interchangeably, and all refer to single domain antibodies that specifically recognize and bind to CD70. Single domain antibodies are variable regions of heavy chain antibodies. Typically, single domain antibodies comprise three CDRs and four FRs. Preferably, the single domain antibody of the present description has CDR1 shown by any one of SEQ ID NO: 1-7, CDR2 shown by any one of SEQ ID NO: 8-13 and CDR3 shown by any one of SEQ ID NO: 14-20. Single domain antibodies are the smallest functional antigen binding fragments. Generally, after obtaining an antibody with natural deletion of light chain and heavy chain constant region 1 (CH1), the variable region of antibody heavy chain is cloned to construct a single domain antibody consisting of only one heavy chain variable region.

The described "nanoantibody" refers to an antibody comprising a VHH described herein. It can be a heavy chain antibody as described above, or a multivalent or multispecific antibody containing multiple VHHs, or can be obtained by recombining VHH and antibody Fc (such as CH2 and CH3 or CH2, CH3 and CH4) recombinant antibodies.

Binding molecules comprising two or more single domain antibodies are multivalent single domain antibodies; and binding molecules comprising two or more single domain antibodies with different specificities are multispecific single domain antibodies. Multivalent single domain antibodies or multispecific single domain antibodies are linked to multiple single domain antibodies through linkers. The linker usually consists of 1-15 amino acids selected from G and S.

Heavy chain antibodies and antibodies (traditional four-chain antibodies) herein are distinguished by different combinations of antibodies. Due to the similarity of their structures, the following structural descriptions for antibodies also apply to heavy chain antibodies except for light chains.

The "variable region" or "variable domain" of an antibody refers to the amino-terminal domains of the heavy or light chain of the antibody. The variable domains of the heavy chain and light chain may be referred to as "VH" and "VL", respectively. These domains are usually the most variable parts of the antibodies (relative to other antibodies of the same type) and contain the antigen binding sites.

The term "variable" refers to the fact that certain segments of the variable domains differ extensively in sequence among antibodies. Variable domains mediate antigen binding and define the specificity of a specific antibody to its specific antigen. However, variability is not evenly distributed across all amino acids spanned by the variable domain. On the contrary, it is concentrated in three segments called hypervariable region (HVR) (both in the light chain and the heavy chain variable domains), namely HCDR1, HCDR2 and HCDR3 of heavy chain variable region (CDR1, CDR2 and CDR3 in heavy chain antibodies for short) and LCDR1, LCDR2 and LCDR3 of light chain variable region. The more highly conserved part of the variable domain is called the framework regions (FR). The variable domains of native heavy and light chains each comprise four FR regions (FR1, FR2, FR3, and FR4), largely adopting a β -sheet configuration, connected by three HVRs, which form loops connecting, and in some cases forming part of, the β-sheet structure. The HVR in each chain is hold together by the FR region in close proximity, and contributes to the formation of the antigen binding site of the antibody together with the HVR of the other chain. Generally, the structure of the light chain variable region is FR1-LCDR1-FR2-LCDR2-FR3-LCDR3-FR4, and the structure of the heavy chain variable region is FR1-HCDR1-FR2-HCDR2-FR3-HCDR3-FR4. The constant domain is not directly involved in the binding of antibody to antigen, but exhibits a variety of effector functions, such as the involvement of antibody in antibody-dependent cell-mediated toxicity. There are a variety of variable region labeling schemes for antibodies, including: Chothia, Kabat, IMGT, and Contact. The present description exemplarily uses the IMGT labeling scheme.

An "Fc region" (fragment crystallizable region) or "Fc domain" or "Fc" refers to the C-terminal region of the heavy chain of an antibody that mediates the binding of the immunoglobulin to host tissues or factors, comprising binding to Fc receptors located on various cells of the immune system (e.g., effector cells) or to the first component (Clq) of the classical complement system. In IgG, IgA and IgD antibody isotypes, the Fc region is composed of two identical protein fragments from the CH2 domain and CH3 domain of two heavy chains of the antibody; the Fc regions of IgM and IgE comprise three heavy chain constant domains (CH domains 2-4) in each polypeptide chain. Although the boundary of the Fc region of an immunoglobulin heavy chain may vary, the Fc region of human IgG heavy chain is usually defined as a sequence segment from the amino acid residue of heavy chain position C226 or P230 to the carboxyl terminus, which is numbered according to the EU index, as in Kabat. As used herein, the Fc region may be a native sequence Fc or a variant Fc.

An "antibody fragment" comprises a portion of an intact antibody, preferably the antigen binding and/or the variable region of the intact antibody. The antibody fragment is preferably an antigen binding fragment of the antibody. Examples of antibody fragments include Fab, Fab', F(ab'), F(ab')2, Fd, Fv fragments and disulfide-linked Fv; diabodies; linear antibodies; single-chain antibody molecules, scFv-Fc fragment; multispecific antibodies formed from antibody fragments; and any fragment that should be able to increase the half-life by chemical modification or by incorporation into liposomes. Antigen-binding fragments can be prepared by a variety of techniques including, but not limited to, proteolytic digestion of intact antibody proteins and generation by expression from host cells comprising antigen-binding fragments.

"Fv" is the minimum antibody fragment which comprises a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site. "Single-chain Fv" also abbreviated as "sFv" or "scFv", which are antibody fragments that comprise the VH and VL antibody domains connected into a single polypeptide chain. Preferably, the sFv polypeptide further comprises a polypeptide linker between the VH and VL domains which enables the sFv to form the desired structure for antigen binding. For heavy chain antibodies or nanoantibodies, the scFv is the VHH.

The term "monoclonal antibody" as used herein refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present description may be made by a variety of techniques, including, for example, the hybridoma method, phage-display technologies, recombinant DNA methods, and technologies for producing human or human-like antibodies in animals that have parts or all of the human immunoglobulin loci or genes encoding human immunoglobulin sequences, single-cell sequencing methods.

The monoclonal antibodies herein specifically comprise "chimeric" antibodies in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity.

"Humanized" forms of non-human (e.g., murine) antibodies are chimeric antibodies that contain minimal sequence derived from non-human immunoglobulin. Therefore, "humanized antibodies" generally refer to non-human antibodies that have had the variable-domain framework regions swapped for sequences found in human antibodies. Usually in a humanized antibody, the entire antibody (except CDRs) is encoded by or identical to a human derived polynucleotide (except CDRs). The CDRs, some or all of which are encoded by nucleic acids originating in a non-human organism, are grafted into the β -sheet framework of a human antibody variable region to create an antibody, the specificity of which is determined by the engrafted CDRs. The production methods of such antibodies are well known in the art, such as using mice with genetically engineered immune systems. In the present description, antibodies, single domain antibodies, heavy chain antibodies, etc. all include humanized variants of the antibodies.

A "human antibody" is an antibody that possesses an amino-acid sequence corresponding to that of an antibody produced by a human and/or has been made using any of the techniques for making human antibodies as disclosed herein. This definition of human antibodies clearly excludes a humanized antibodies that contain non-human antigen binding residues. Human antibodies can be generated using a variety of techniques known in the art, including phage display libraries.

In some embodiments, the present description also provides a nanoantibodies, heavy chain antibodies, antibodies or antigen binding fragment thereof that bind to the same epitope of human CD70 in the antigenic splicing region of any anti-CD70 nanoantibody of the present description (e.g., single domain antibody VHH), that is, nanoantibodies, heavy chain antibodies, antibodies or antigen binding fragment thereof that can cross-compete with the antigen-binding region of any nanoantibody of the present description for binding to CD70.

In the present description, the single domain antibody of the description has CDR1 as shown by any of SEQ ID NO: 1-7, CDR2 as shown by any of SEQ ID NO: 8-13 and CDR3 as shown by any of SEQ ID NO: 14-20. Preferably, the anti-CD70 single domain antibody comprises CDR1, CDR2 and CDR3 shown by any group of (a)-(h).
(a) CDR1 which sequence is shown by SEQ ID NO: 1, CDR2 which sequence is shown by SEQ ID NO:8, and CDR3 which sequence is shown by SEQ ID NO: 14;
(b) CDR1 which sequence is shown by SEQ ID NO:2, CDR2 which sequence is shown by SEQ ID NO:8, and CDR3 which sequence is shown by SEQ ID NO: 14;
(c) CDR1 which sequence is shown by SEQ ID NO:3, CDR2 which sequence is shown by SEQ ID NO:8, and CDR3 which sequence is shown by SEQ ID NO: 15;
(d) CDR1 which sequence is shown by SEQ ID NO:4, CDR2 which sequence is shown by SEQ ID NO:9, and CDR3 which sequence is shown by SEQ ID NO: 16;
(e) CDR1 which sequence is shown by SEQ ID NO:5, CDR2 which sequence is shown by SEQ ID NO:10, and CDR3 which sequence is shown by SEQ ID NO:14;
(f) CDR1 which sequence is shown by SEQ ID NO:6, CDR2 which sequence is shown by SEQ ID NO:11, and CDR3 which sequence is shown by SEQ ID NO:18;
(g) CDR1 which sequence is shown by SEQ ID NO:6, CDR2 which sequence is shown by SEQ ID NO:12, and CDR3 which sequence is shown by SEQ ID NO:19;
(h) CDR1 which sequence is shown by SEQ ID NO:7, CDR2 which sequence is shown by SEQ ID NO:13, and CDR3 which sequence is shown by SEQ ID NO:20;

FR1, FR2, FR3 and FR4 of the anti-CD70 single domain antibody described herein can be independently selected from FR1, FR2, FR3 and FR4 of the single domain antibody shown by any of SEQ ID NO:21-28. Preferably, the amino acid sequence of the anti-CD70 single domain antibody is shown by any one of SEQ ID NO:21-28.

When a single domain antibody is linked to a heavy chain constant region, the nanoantibody is a heavy chain antibody comprising the single domain antibody described herein. The heavy chain constant region may be a constant region of a camelid heavy chain antibody, comprising CH2 and CH3. Preferably, the antibody constant region is derived from: the constant region of any of IgG1, IgG2, IgG3, IgG4, IgA, IgM, IgE and IgD, more preferably derived from the constant region of any of IgG1, IgG2, IgG3, IgG4. In one or more embodiments, the heavy chain constant region is CH2 and CH3 of human IgG Fc, shown by such as CH2 and CH3 of IgG1, which is shown by SEQ ID NO:37.

The CD70 binding molecules described herein are monovalent or multivalent nanoantibodies or single-domain antibodies, or multispecific nanoantibodies or single-domain antibodies, comprising one, two or more of anti-CD70 nanoantibodies or single-domain antibodies described herein. The multi specificity can be against CD70 and another antigen, or it can be against two different epitopes of CD70.

The present description also comprises the antibody derivatives and analogues. "Derivatives" and "analogues" refer to polypeptides that basically maintain the same biological function or activity of the antibody of the present description. The derivatives or analogues of the present description may be polypeptides formed from (i) a polypeptide with a substituent group in one or more amino acid residues, or (ii) a polypeptide formed from fusion of a mature polypeptide with another compound, such as a compound that extends the half-life of the polypeptide, such as polyethylene glycol, or (iii) a polypeptide formed by fusing an additional amino acid sequence to this polypeptide sequence (such as a leader sequence or a secretory sequence, or a sequence or prokaryotic sequence used for purifying this polypeptide, or a fusion protein formed with a 6His tag). According to the teaching herein, these derivatives and analogues belong to common sense known to those skilled in the art.

Without substantially affecting the activity of the antibody, those skilled in the art may change one or more (for example, 1, 2, 3, 4, 5, 6, 7, 8, 9 or 10 or more) amino acids in the antibody sequence of the present description to obtain the variant of the antibody or the functional fragment sequence thereof. These variants include (but are not limited to): deletion, insertion, and/or substitution of one or more (usually 1-50, preferably 1-30, more preferably 1-20, and most preferably 1-10) amino acids, and addition of one or more (usually less than 20, preferably less than 10, and more preferably less than 5) amino acids at the C-terminus and/or N-terminus. In this field, conservative substitution with amino acids with similar or similar properties usually does not change the function of the protein. For example, substituting with amino acids having similar properties may be performed in the FR and/or Fc regions. Amino acid residues available for conservative substitution are well known in the art. Such substituted amino acid residues may or may not be encoded by a genetic code. For another example, adding one or more amino acids to the C-terminus and/or N-terminus usually does not change the function of the protein. They are all considered to be included in the scope of the present description.

The variant forms of the antibody described herein include: homologous sequence, conservative variant, allelic variant, natural mutant, induced mutant, protein encoded by DNA that can hybridize with the coding DNA of the antibody of the description under high or low strictness conditions, and polypeptide or protein obtained by using the antiserum of the antibody of the description. In some embodiments, the sequence of the variant of the present description may have at least 95%, 96%, 97%, 98% or 99% identity with its source sequence. The sequence identity described in the description can be measured using sequence analysis software. For example, the computer program BLAST with default parameters, especially BLASTP or TBLASTN. The description also comprises those molecules with variable regions of antibody heavy chain with CDRs, if their CDRs have more than 90% homology (preferably more than 95%, more preferably more than 98%) with the CDRs identified here.

The antibody of the description can be prepared by conventional methods in the art, such as hybridoma technology. The nanoantibody of the description can be prepared by conventional methods in the art, such as phage display technology well known in the art. Alternatively, the antibody or nanoantibody of the present description may be expressed in other cell lines. Suitable mammalian host cells can be transformed with sequences encoding antibody of the description, and then the host cells can be cultured and the antibody purified. Transformation can be carried out using any known method, including, for example, packaging polynucleotides in viruses (or viral vectors) and transducing host cells with the viruses (or vectors). The transformation procedure used depends on the host to be transformed. Methods for introducing heterologous polynucleotides into mammalian cells are well known in the art, including dextran mediated transfection, calcium phosphate precipitation, polybrene mediated transfection, protoplast fusion, electroporation, encapsulation of polynucleotides in liposomes and direct microinjection of DNA into the nucleus. Mammalian cell lines that can be used as hosts for expression are well known in the art, including but not limited to a variety of immortalized cell lines available from the American Typical Culture Collection (ATCC), including but not limited to Chinese hamster ovary (CHO) cells, HeLa cells, baby hamster kidney (BHK) cells, monkey kidney cells (COS), human hepatocellular carcinoma cells (e.g., HepG2), etc.

### CAR

The present description provides a CD70-targeted chimeric antigen receptor (CAR). The CAR comprises an optional signal peptide sequence, an antigen recognition region, namely the anti-CD70 binding molecule described herein, a hinge region, a transmembrane region and an intracellular region. Wherein the intracellular region comprises one or more intracellular costimulation domains and/or one or more intracellular signaling domains. The "hinge region", "transmembrane region" and "intracellular region" herein can be selected from the sequences of the hinge region, transmembrane region and intracellular region in the known CAR-T technology.

Optional signal peptides of CAR can be selected as desired. In general, a signal peptide is a peptide sequence that targets a polypeptide to a desired location in a cell. The signal peptide targets the polypeptide to the secretory pathway of the cell and will allow integration and anchoring of the polypeptide to the lipid bilayer; the signal peptide may also be a membrane localized signal peptide. Exemplary, CD8 signal peptide, CD28 signal peptide, CD4 signal peptide or light chain signal peptide, the sequences of which are within the knowledge of those skilled in the art. The CD8 signal peptide suitable for the present description can be various human CD8 signal peptide sequences commonly used for CAR in the art. In certain embodiments, the amino acid sequence of the human CD8 signal peptide comprises the sequence as shown by SEQ ID NO:38.

The hinge region of a chimeric antigen receptor is located between the extracellular antigen-binding region and the transmembrane region, and the hinge region is an amino acid segment that is normally present between two domains of a protein and allows for flexibility of the protein and movement of the two domains relative to each other. The hinge region may be the hinge region of a naturally occurring protein or a portion thereof. The hinge regions of antibodies (such as IgG, IgA, IgM, IgE or IgD antibodies) can also be used in the chimeric antigen receptors described herein. Non-naturally occurring peptides can also be used as the hinge region of the chimeric antigen receptors described herein. Exemplary, the hinge region of the CAR is selected from the group consisting of: CD8α hinge region, IgD hinge region, IgG1 Fc CH2CH3 hinge region or IgG4 Fc CH2CH3 hinge region, and its sequence being within the knowledge of those skilled in the art. The CD8α hinge region suitable for the present description can be various human CD8α hinge region sequences commonly used for CAR in the art. In certain embodiments, the human CD8α hinge region comprises the sequence as shown by SEQ ID NO:39.

The transmembrane region of the chimeric antibody receptor may form an α helix, a complex of more than one α helix, a β barrel, or any other stable structure capable of spanning the domain cellular phospholipid bilayer. Transmembrane regions can be of natural or synthetic origin. The transmembrane region may be selected from the group consisting of transmembrane regions of the following proteins: CD3ε, CD4, CD5, CD8α, CD9, CD16, CD22, CD28, CD33, CD37, CD45, CD64, CD80, CD86, CD134, CD137, CD154, T cell receptor α, β, or ζ chain of a body. The CD8α transmembrane region suitable for the present description can be various human CD8α transmembrane region sequences commonly used for CAR in the art. In certain embodiments, the amino acid sequence of the human CD8α signal peptide comprises the sequence as shown by SEQ ID NO:40.

The intracellular signaling region (or intracellular signaling region) is responsible for the activation of at least one normal effector function of immune effector cells expressing chimeric antigen receptors. For example, the effector function of T cells can be cell lysis activity or helper activity, including cytokine secretion. While it is often possible to utilize the entire intracellular signaling domain, in many cases the use of the entire chain is unnecessary. To the extent that truncated portions of the intracellular signaling region are used, such truncated portions can be used in place of the intact chain as long as they transduce effector function signals. Thus, an intracellular signaling region includes any truncated form of an intracellular signaling region sufficient to transduce an effector function signal. The intracellular signaling domain of CAR can be selected according to needs, including but not limited to those derived from at least one of CD3ζ, FcRy (FCER1G), FcRβ (FcsRib), CD3γ, CD3δ, CD3ε, CD5, CD22, CD79a, CD79b and CD66d intracellular signaling domain. Preferably, the intracellular signaling region is derived from the intracellular signaling region of human CD3ζ. Further, the human CD3ζ intracellular signal region has the amino acid sequence shown by SEQ ID NO:41.

In addition to stimulation by antigen-specific signals, many immune effector cells require costimulation to promote cell proliferation, differentiation, and survival, as well as to activate the effector functions of the cells. A "costimulatory domain" can be the cytoplasmic portion of a costimulatory molecule. The term "costimulatory molecule" refers to an associated binding chaperone on an immune cell (such as a T cell), that binds specifically to a costimulatory ligand so that the immune cell mediates a costimulatory response, such as, but not limited to, proliferation and survival. Suitable intracellular costimulatory domains can be selected according to needs, including intracellular domains with costimulatory signal molecules, such as those derived from 4-1BB, CARD11, CD2, CD7, CD27, CD28, CD30, CD40, CD54, CD83, OX40, CD137, CD134, CD150, CD152, CD223, CD270, PD-L2, PD-L1, CD278, DAP10, LAT, NKD2C, SLP76, TRIM, FcεRIγ, MyD88, and at least one of the intracellular domains of 41BBL. In some embodiments, the amino acid sequence of the 4-1BB costimulatory domain comprises the sequence shown by SEQ ID NO:42.

The above-mentioned parts that form the chimeric antigen receptor of the present invention, such as the CD8 signal peptide, the anti-MSLN nanoantibody, the CD8 hinge region, the CD28 transmembrane region, the CD28 costimulatory domain, the CD3ζ intracellular signaling domain, etc., can be linked directly to each other or through linker sequences. The linker sequence may be one well known in the art and suitable for use with antibodies, such as a G-and-S-containing linker sequence. Typically, linkers contain one or more tandemly repeated motifs. For example, the motif can be GGGS, GGGGS, SSSSG, GSGSA and GGSGG. Preferably, the motifs are contiguous in linker sequences with no intervening amino acid residues between the repeats. Linker sequences can consist of 1, 2, 3, 4 or 5 repeat motifs. The length of the linker can be 3 to 25 amino acid residues, such as 3 to 15, 5 to 15, or 10 to 20 amino acid residues. In certain embodiments, the linker sequence is a polyglycine linker sequence. The number of glycines in the linker sequence is not particularly limited, usually 2 to 20, such as 2 to 15, 2 to 10, 2 to 8. In addition to glycine and serine, the linker may further comprise other known amino acid residues, such as alanine (A), leucine (L), threonine (T), glutamic acid (E), phenylalanine (F), arginine (R), glutamine (Q), etc. In certain embodiments, the linker sequence is a (GGGGS)n linkage, where n is an integer from 1 to 5.

In an exemplary embodiment, the CAR comprises CD8 signal peptide, anti-CD70 nanoantibody or antigen-binding fragment thereof described herein, CD8α hinge region, CD28α transmembrane region, CD3ζ intracellular signaling domain and 4-1BB costimulatory domain in sequence from N-terminus to C-terminus. In specific embodiments, an exemplary CAR having the above structure is shown by any of SEQ ID NOs: 29-36.

It should be understood that in gene cloning operation, it is often necessary to design a suitable cleavage site, which is bound to introduce one or more incoherent residues at the ends of the expressed amino acid sequence, and this does not affect the activity of the sequence. Also for constructing fusion proteins, facilitating the expression of recombinant proteins, obtaining recombinant proteins that are automatically secreted outside the host cell, or facilitating the purification of recombinant proteins, it is often necessary to add some amino acids to the N-terminus, C-terminus, or other suitable regions within the recombinant protein, for example, including but not limited to, suitable linker peptides, signal peptides, leader peptides, terminal extensions, etc. Therefore, the amino terminus or carboxyl terminus of the CAR of the present description may also comprise one or more polypeptide fragments as protein tags. Any suitable tag may be used herein. For example, the tags may be FLAG, HA, HA1, c-Myc, Poly-His, Poly-Arg, Strep-TagII, AU1, EE, T7, 4A6, ε, B, gE and Ty1. There tags can be used to purify proteins.

The antigen recognition region in the CAR of the present invention may be a variant of the aforementioned anti-CD70 nanoantibodies or functional fragment sequences thereof. In addition, other parts of the CAR can also undergo sequence changes, and the resulting mutant has at least 80%, preferably at least 85%, preferably at least 90%, preferably at least 95%, preferably at least 97% sequence identity with the CAR and retaining the biological activity of the CAR (such as activated T cells). The sequence identity between two aligned sequences can be calculated using, for example, NCBI's BLASTp.

These mutants further comprise the amino acid sequence having one or more mutations (insertions, deletions, or substitutions) in the amino acid sequence of the CAR of any embodiment while still retaining the biological activity of the CAR. The more mutations usually refer to within 1-10, such as 1-8, 1-5 or 1-3. Substitutions are preferably conservative substitutions. For example, in this field, conservative substitutions with amino acids with similar or identical properties usually do not change the function of the protein. The "amino acids with similar or identical properties" includes, for example, families of amino acid residues with similar side chains. These families comprise amino acids with basic side chains (e.g., lysine, arginine, histidine), amino acids with acidic side chains (e.g., aspartic acid, glutamic acid), amino acids with uncharged polar side chains (e.g., glycine, asparagine, glutamine, serine, threonine, tyrosine, cysteine), amino acids with non-polar side chains (e.g., alanine, valine, leucine, isoleucine, proline, phenylalanine, methionine, tryptophan), amino acids with β-branched side chains (e.g., threonine, valine, isoleucine), and amino acids with aromatic side chains (e.g., tyrosine, phenylalanine, tryptophan, histidine). Therefore, substitutions of one or more positions in a polypeptide of the present description with another amino acid residue from the same side chain will not materially affect its activity.

### Nucleic acid

The present description also provides polynucleotides encoding the above antibody or CAR thereof. The polynucleotide of the description can be in the form of DNAs or RNAs. DNAs comprise cDNAs, genomic DNAs, or synthetic DNAs. DNAs can be single stranded DNAs or double stranded DNAs. DNAs can be coding or noncoding strand DNAs. The present description also comprises degenerate variants of the polynucleotide sequence encoding the fusion protein, i.e., nucleotide sequences encoding the same amino acid sequence but with different nucleotide sequences.

Therefore, the present description also relates to polynucleotides that hybridize with the above polynucleotide sequences and have at least 50%, preferably at least 70%, more preferably at least 80% identity between the two sequences. The present description particularly relates to polynucleotides that can hybridize with the polynucleotides of the description under strict conditions. In the present description, "strict conditions" refer to: (1) hybridization and elution at lower ionic strength and higher temperature, such as 0.2 × SSC, 0.1%SDS, 60 °C; or (2) addition of denaturants during hybridization, such as 50% (v/v) formamide, 0.1% calf serum/0.1% Ficoll, 42 °C, etc; or (3) hybridization that occurs only when the identity between two sequences is at least more than 90%, or preferably, more than 95%. Moreover, the polypeptides encoded by hybridizable polynucleotides have the same biological functions and activities as mature polypeptides.

The nucleotide full-length sequence of the antibody of the present description or fragment thereof can usually be obtained by PCR amplification method, recombination method or artificial synthesis method. A feasible method is to synthesize relevant sequences by artificial synthesis, especially with short fragment length. Usually, fragments with very long sequence can be obtained by synthesizing several small fragments first and then connecting them. In addition, the coding sequence of the heavy chain and the expression tag (such as 6His) can also be fused together to form a fusion protein. The sequence of CAR can also be obtained as above. Alternatively, the sequence of each part of the CAR (signal peptide, antigen recognition region, hinge region, transmembrane region or intracellular region) can be obtained as above and then ligated to obtain the full length of the CAR.

Once the relevant sequences are obtained, they can be obtained in large quantities by recombination. They are related sequences usually cloned into vectors, transfered into cells, and then isolated from the proliferated host cells by conventional methods. The biomolecules (nucleic acids, proteins, etc.) according to the present description comprise biomolecules in isolated form. At present, the DNA sequence encoding the protein (or fragment thereof, or derivative thereof) of the present disclosure can be obtained completely through chemical synthesis. This DNA sequence can then be introduced into various existing DNA molecules (or such as vectors) and cells known in the art. In addition, mutations can also be introduced into the protein sequence of the present description through chemical synthesis. Each part of the CAR can be cloned sequentially into a vector or can be integrated into a full-length CAR and then cloned.

The present description also relates to nucleic acid constructs containing the polynucleotide sequences described herein, and one or more regulatory sequences operably linked to these sequences. The polynucleotide sequence described herein can be manipulated in a variety of ways to ensure expression of the antibody or CAR. The nucleic acid construct can be manipulated according to the difference or requirements of the expression vector prior to insertion of the nucleic acid construct into the vector. Techniques that utilize recombinant DNA methods to alter polynucleotide sequences are known in the art.

Regulatory sequences can be suitable promoter sequences. The promoter sequence is usually operably linked to the coding sequence of the protein to be expressed. The promoter can be any nucleotide sequence that shows transcriptional activity in the host cell of choice, comprising mutated, truncated, and hybrid promoters, and can be derived from genes encoding extracellular or intracellular polypeptides homologous or heterologous to the host cell. An example of a suitable promoter is the immediate early cytomegalovirus (CMV) promoter sequence. The promoter sequence is a strong constitutive promoter sequence capable of driving high-level expression of any polynucleotide sequence operably linked thereto. Another example of a suitable promoter is elongation growth factor-1α (EF-1α). However, other constitutive promoter sequences can also be used, including but not limited to the early stage of simian virus 40 (SV40) promoter, mouse mammary tumor virus (MMTV), human immunodeficiency virus (HIV) long terminal repeat (LTR) promoter, MoMuLV promoter, avian leukemia virus promoter, Epstein-Barr virus immediate early promoter, Rous sarcoma virus promoters, as well as human gene promoters, such as, but not limited to, actin promoter, myosin promoter, heme promoter, and creatine kinase promoter. Furthermore, the use of inducible promoters may also be considered. The use of inducible promoters provides a molecular switch capable of turning on expression of a polynucleotide sequence operably linked to the inducible promoter when expression is required and turning off expression when expression is undesirable. Examples of inducible promoters include but are not limited to metallothionein promoters, glucocorticoid promoters, progesterone promoters, and tetracycline promoters.

The regulatory sequence can also be a suitable transcription termination sequence, a sequence recognized by a host cell to terminate transcription. A terminator sequence is operably linked to the 3' terminal of the nucleotide sequence encoding the polypeptide. Any terminator that is functional in the host cell of choice can be used in the present description. The regulatory sequence can also be a suitable leader sequence, an untranslated region of the mRNA important for translation by the host cell. A leader sequence is operably linked to the 5' terminal of the nucleotide sequence encoding the polypeptide. Any terminator that is functional in the host cell of choicecan be used in the present description.

In some embodiments, the nucleic acid construct described herein is a vector, such as a cloning vector, an expression vector and an integration vector. Expression of the present polynucleotide sequences is generally achieved by operably ligating the polynucleotide sequence of the description to an expression vector. A typical cloning vector comprises transcriptional and translational terminators, initiation sequences and a promoter useful for regulating the expression of the desired nucleic acid sequence. Integrating vectors contain components for integrating the target sequence into the genome of the cell. These vectors can be used to transform appropriate host cells to enable them to express proteins. Vectors usually contain sequences for plasmid maintenance and for cloning and expressing exogenous nucleotide sequences. The sequences (collectively referred to as "flanking sequence" in some embodiments) generally comprises one or more of the following nucleotide sequences: promoter, one or more enhancer sequences, replication origin, transcription termination sequence, complete intronic sequence comprising donor and receptor splice sites, sequence encoding leader sequence for polypeptide secretion, ribosome binding site, polyadenylation sequence, a multi-linker region for inserting nucleic acids encoding antibodies to be expressed and an optional marker element.

In addition, the type of vector is not limited, for example, plasmids, phagemids, phage derivatives, animal viruses, and cosmids, which may vary depending on the host cell to be introduced. Viral vector technology is well known in the art and described, for example, in Sambrook et al. (2001, Molecular Cloning: A Laboratory Manual, Cold Spring Harbor Laboratory, New York) and other handbooks of virology and molecular biology. Viruses that can be used as vectors include, but are not limited to, retroviruses, adenoviruses, adeno-associated viruses, herpesviruses, and lentiviruses.

To assess the expression of a CAR polypeptide or portion thereof, the vector introduced into the cell can also comprise either or both of a selectable marker gene or a reporter gene to facilitate identification and selection of expressing cells accessed from a cluster of transfected or infected cells by the viral vector.

### Cell

The host cells suitable for introducing the nucleic acid constructs described herein can be prokaryotic cells, such as bacterial cells; or lower eukaryotic cells, such as yeast cells; or higher eukaryotic cells, such as mammalian cells, especially immune cells, preferably the immune effector cells. Representative examples are: Escherichia coli, Streptomyces; bacterial cells of Salmonella typhimurium; fungal cells such as yeast; insect cells of Drosophila S2 or Sf9; animal cells of CHO, COS7, 293 cells, etc.

Immune effector cells are immune cells that can perform immune effector functions. In some embodiments, the immune effector cells express at least FcγRIII and perform ADCC effector functions. Examples of immune effector cells that mediate ADCC include peripheral blood mononuclear cells (PBMC), natural killer cells (NK), monocytes, cytotoxic T cells, neutrophils, and eosinophils. Preferably, the immune effector cells are selected from: at least one of immune cells cultured and differentiated from pluripotent stem cells or embryonic stem cells, T lymphocytes, NK cells, peripheral blood mononuclear cells (PBMC) and hematopoietic stem cells. More preferably, the immune effector cells are T lymphocytes (same as T cells). In some embodiments, T cells can be CD4+/CD8-, CD4-/CD8+, CD4+/CD8+, CD4-/CD8-, or combinations thereof. In some embodiments, the T cells produce IL-2, IFN and/or TNF when expressing chimeric antigen receptors and binding to target cells. In some embodiments, CD8+T cells lyse antigen-specific target cells when expressing chimeric antigen receptors and binding to target cells.

T cells suitable for use in the present description can be various types of T cells from various sources. For example, T cells can be derived from PBMCs of patients with B-cell malignancies. In certain embodiments, the obtained T cells can be first stimulated and activated with an appropriate amount (for example, 30-80 ng/ml, such as 50 ng/ml) of CD3 antibodies, and then cultured in a medium containing an appropriate amount of IL2 (for example, 30 to 80 IU/ml, such as 50 IU/ml) for later use.

Methods for introducing nucleic acids or vectors into mammalian cells are known in the art, and the vectors may be transferred into cells by physical, chemical or biological methods. When the host is a prokaryote such as *Escherichia coli,* competent cells that can absorb DNA can be harvested after the exponential growth period and treated with CaCl₂ method, the steps of which are well known in the art. When the host is eukaryote, the following DNA transfection methods can be selected: calcium phosphate coprecipitation method, conventional mechanical methods such as microinjection, electroporation, liposome packaging, etc. In some embodiments, transduced or transfected immune effector cells proliferate ex vivo after introducing nucleic acids or vectors.

The obtained transformants can be cultured by conventional methods to express the antibody or CAR encoded by the gene of the present description. According to the host cells used, the medium used in the culture can be selected from various conventional media. Culture is performed under conditions suitable for host cell growth. When the host cells grow to the appropriate cell density, the selected promoters are induced by appropriate methods (such as temperature conversion or chemical induction), and the cells are cultured for another period of time.

The polypeptide in the above method can be expressed inside the cell, on the cell membrane, or secreted outside the cell. If necessary, the recombinant protein can be separated and purified by various separation methods using its physical, chemical, and other characteristics. These methods are familiar to those skilled in the art. Examples of these methods include but are not limited to: conventional renaturation treatment, treatment with protein precipitant (salting-out method), centrifugation, permeation, ultra-treatment, ultra-centrifugation, molecular sieve chromatography (gel filtration), adsorption chromatography, ion exchange chromatography, high-performance liquid chromatography (HPLC) and other various liquid chromatography technologies and combinations of these methods.

### Uses and Methods

By constructing a nanoantibody library, the inventors screened nanoantibody and variants thereof that could bind CD70. The binding of these antibodies to antigen was verified by protein-level binding assays, affinity assays, competitive blocking experiments, and tissue cross-reactions. Utilizing these nanoantibodies, the inventors developed CARs and CAR-T cells, and molecular and cellular level experiments confirmed that the CAR-T cells have strong immune functionality, better expression of CD107a, IFN-γ and IL-2 secretion and specific killing function on target cells, the drug effect in vivo is remarkable.

All aspects of the nanoantibodies, CAR, coding sequences, nucleic acid constructs, and cells described herein can be used in the preparation of medicaments for the prevention or treatment of the various conditions and diseases described herein that are associated with CD70 expression, which refer to the diseases directly or indirectly caused by the abnormal expression of CD70, and usually refer to the diseases caused by the overexpression of CD70, such as cancer, including but not limited to: renal cell carcinoma, acute myelogenous leukemia, non-Hodgkin Gold lymphoma, multiple myeloma, mantle cell lymphoma, diffuse large cell lymphoma, follicular lymphoma, pancreatic cancer, breast cancer, glioblastoma.

The present description also comprises a type of cell therapy comprising expressing a CAR described herein in immune cells (e.g., T cells), and administering to a recipient in need thereof a therapeutically effective amount of the cells capable of killing tumor cells in the recipient. Compared with antibody therapies, CAR-T cells are able to replicate in vivo, producing long-term persistence that can lead to sustained tumor control. The anti-tumor immune response caused by CAR-T cells can be an active or passive immune response. Additionally, a CAR-mediated immune response can be part of an adoptive immunotherapy step, in which CAR-T cells induce an immune response specific for the antigen-binding portion of the CAR.

The antibodies, nucleic acids or CAR-modified cells of the present description can be administered alone or as a pharmaceutical composition in combination with a diluent and/or with other components such as relevant cytokines or cell populations. In this regard, pharmaceutical compositions can be prepared by admixing the active agent having the desired degree of purity, optionally with a pharmaceutically acceptable carrier, as a lyophilized formulation or as an aqueous solution. Pharmaceutically acceptable carriers are non-toxic to recipients at the dosages and concentrations employed and can include at least one of buffering agents (e.g., neutral buffered saline, sulfate buffered saline), antioxidants, preservatives, isotonic agents, stabilizing agents, a chelating agent (such as EDTA or glutathione), an adjuvant (such as aluminum hydroxide) and a surfactant. Furthermore, in order for pharmaceutical compositions to be useful for in vivo administration, they must be sterile. Pharmaceutical compositions can be rendered sterile by filtration through sterile filtration membranes.

In some embodiments, the pharmaceutical composition may contain at least one additive of cytotoxic agents, chemotherapeutic agents, cytokines, immunosuppressants, growth inhibitors, and active agents as required for the particular indication being treated. The specific addition amount of additives can be adjusted according to actual needs.

The pharmaceutical composition of the present invention can be administered in an amount of "immunologically effective amount", "anti-tumor effective amount", "tumor-inhibitory effective amount" or "therapeutic amount". "Treatment" refers to the subject accepting the treatment scheme described herein to achieve at least one positive treatment effect (for example, a reduced number of cancer cells, a reduced tumor volume, a reduced rate of cancer cells infiltrating into peripheral organs, or a redeuced rate of tumor metastasis or tumor growth). When an "immunologically effective amount", "anti-tumor effective amount", "tumor-inhibitory effective amount" or "therapeutic amount" is indicated, the precise amount of the composition of the present description to be administered can be determined by a physician, taking into account patients' (subjects') age, weight, tumor size, extent of infection or metastasis, and individual differences in the condition. Generally, pharmaceutical compositions comprising T cells described herein may be administered at a dose of 10⁴ to 10⁹ cells/kg body weight, preferably 10⁵ to 10⁶ cells/kg body weight. T cell compositions can also be administered multiple times at these dosages. Cells can be administered using infusion techniques well known in immunotherapy (see, e.g., Rosenberg et al., New Eng. J. of Med. 319:1676, 1988). The optimal dosage and treatment regimen for a particular patient can be readily determined by one skilled in the medical field by monitoring the patient for signs of disease and adjusting treatment accordingly.

Administration of compositions may be in any convenient manner, including by spraying, injection, swallowing, infusion, implantation or transplantation. The compositions as used herein can be administered to a patient subcutaneously, intradermally, intratumorally, intranodally, intraspinally, intramuscularly, by intravenous injection, or intraperitoneally. In one embodiment, the T cell composition of the invention is administered to the patient by intradermal or subcutaneous injection. In another embodiment, the T cell composition of the present description is preferably administered by intravenous injection. The T cell composition can be injected directly into tumors, lymph nodes, or sites of infection.

In some embodiments of the description, the CAR-T cells or compositions thereof in the present description can be combined with other therapies known in the art. Such therapies include, but are not limited to, chemotherapy, radiotherapy, and immunosuppressants. For example, treatments may be combined with radiotherapy or chemotherapy agents known in the art to treat mesothelin-mediated diseases.

Herein, "anti-tumor effect" refers to a biological effect, which can be expressed by a reduction in tumor volume, a reduction in the number of tumor cells, a reduction in the number of metastases, an increase in life expectancy, or an improvement in various physiological symptoms related to cancer.

"Patient", "subject", "individual" and the like are used interchangeably herein to refer to a living organism, such as a mammal, that can elicit an immune response. Examples include, but are not limited to, humans, dogs, cats, mice, rats, and transgenic species thereof.

The present description is described in further detail with reference to the following experimental examples. These examples are provided for illustrative purposes only and are not intended to be limiting unless otherwise specified. Accordingly, the present description should in no way be construed as limited to the following examples, but should be construed to include any and all changes that become apparent in light of the teachings provided herein. Unless otherwise specified, the methods and materials used in the examples are conventional materials and methods in the art.

### Diagnosis, detection, and kit

The binding molecule of the present description can be used in assays due to its high avidity with CD70, such as binding assays to detect and/or quantify CD70 expressed in tissues or cells. Binding molecules such as single domain antibodies can be used in further studies investigating the function of CD70 in disease. The methods for detecting CD70 are roughly as follows: obtaining cell and/or tissue samples; and detecting the level of CD70 in the sample.

The CD70 binding molecule of the invention can be used for diagnostic purposes to detect, diagnose or monitor diseases and/or conditions associated with CD70. The present description provides the detection of the presence of CD70 in a sample using a classical immunohistological method known to those skilled in the art. Detection of CD70 can be performed in vivo or in vitro. Examples of methods suitable for detecting the presence of CD70 include ELISA, FACS, RIA, and the like.

For diagnostic uses, binding molecules such as single domain antibodies are usually labeled with detectable labeling groups. Suitable labeling groups include (but are not limited to): radioisotopes or radionuclides (e.g., 3H, 14C, 15N, 35S, 90Y, 99Tc, 111In, 1251, 1311), fluorescent groups (e.g., FITC, rhodamine, lanthanide phosphors), enzymatic groups (e.g., horseradish peroxidase, β-galactosidase, luciferase, alkaline phosphatase), chemiluminescent groups, biotinyl groups, or predetermined polypeptide epitopes recognized by secondary reporters (e.g., leucine zipper pair sequences, binding sites for secondary antibodies, metal binding domains, epitope tags), MRI (magnetic resonance imaging) or CT (computed tomography) contrast agents. Various methods for labeling proteins are known in the art and can be used to carry out the present description.

Another aspect of the present description provides a method for detecting the presence of a test molecule that competes with an antibody of the present description to bind CD70. An example of one such assay would involve detecting the amount of free antibody in a solution containing an amount of CD70 in the presence or absence of the test molecule. An increase in the amount of free antibody (i.e., antibody that does not bind CD70) will indicate that the test molecule can compete with the antibody for binding to CD70. In one embodiment, the antibody is labeled with a labeling group.Alternatively, the test molecule is labeled, and the amount of free test molecule is monitored in the presence or absence of the antibody. The present description also provides a detection kit for detecting CD70 level. The kit comprises an antibody that recognizes CD70 protein, a lysis medium for dissolving samples, and general reagents and buffers required for detection, such as various buffers, detection markers, detection substrates, etc. The detection kit can be an in vitro diagnostic device.

The present description is further illustrated in the following examples which are for illustrative purpose only and do not limit the scope of the present description. It should be understood that these examples are merely illustrative and are not intended to limit the scope of the present description. Unless otherwise specified, the methods and materials used in the examples are conventional materials and methods in the art.

### EXAMPLE

Example 1: Construction and eukaryotic expression of recombinant human CD70 protein expression vector

### 1. The synthesis of the gene sequence of the 39th to 193rd amino acid interval of CD70 and the construction of the expression vector of the CD70 protein.

The amino acid sequence from the position 39 to the position 193 of CD70 (uniprot accession No: P32970-1) were imported into the online codon optimization tool (http://www.jcat.de/#opennewwindow) to obtain the codon-optimized nucleic acid sequence, then the gene sequence was obtained by chemical synthesis, and the coding sequence of alpaca IgG1-Fc (amino acid sequence shown by SEQ ID NO: 51) was added to the 3' end of the gene sequence. Through molecular cloning, the spliced product was cloned into pCDNA3.1 (Thermo) with TaKaRa seamless cloning kit to obtain an expression vector.

### 2.Expression, purification and activity identification of recombinant human CD70 protein.

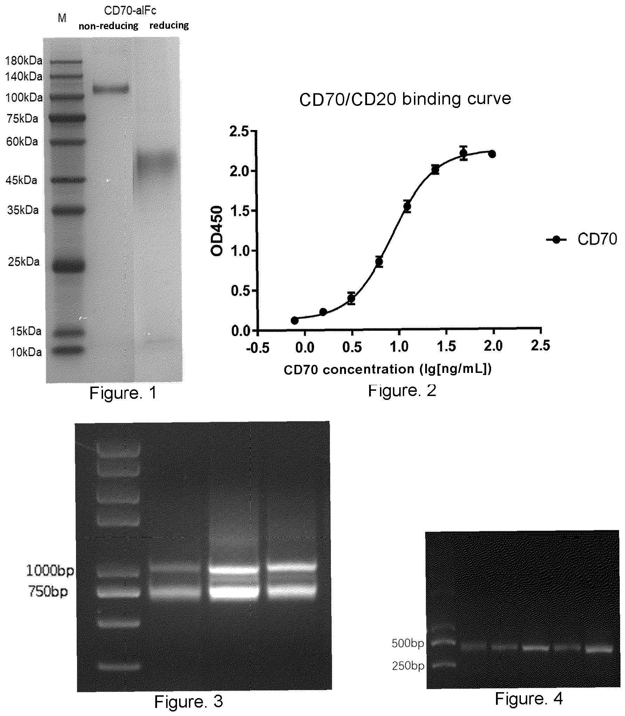

Five days after the obtained expression vector was transfected into 293T cells (ATCC), the culture supernatant was collected, and the recombinant human CD70-alFc protein was purified with AKTA explorer 100 (GE). Due to glycosylation modification and other reasons, the size of the recombinant human CD70-alFc protein was shown to be about 50 kilodaltons after reducing SDS-PAGE electrophoresis and Coomassie brilliant blue staining. The results are shown by Figure 1.

ELISA activity identification of recombinant human CD70-alFc protein with commercial CD27: recombinant human CD70-alFc protein was coated in a 4-fold gradient dilution package in an ELISA plate, the first well was coated with 100 ng, and sequentially diluted 4-fold (100 uL/well), incubated at 4°C overnight, washed 3 times with 200 uL PBST the next day, and blocked with 1% BSA/PBS for 1 hour. The blocking solution was removed, 100 µL 0.1 µg/mL recombinant human CD27-his protein was added to each well, and incubated at 37°C for 1 hour. After washing 3 times with 200 µL PBST, horseradish peroxidase-labeled rabbit anti-his-tag antibody (100 µL per well) diluted 1:4000 was added, and incubated at 37°C for 1 hour. After washing 3 times with 200 µL PBST, 100 µL TMB chromogen solution was added for development at 37°C for 10 minutes, 100 µL 1M hydrochloric acid was added to stop the reaction, and the absorbance value at OD₄₅₀ was obtained.The analysis results are shown by Figure 2 and Table 1.

**Table 1 analysis results**

| | |
|---|---|
| EC50(ng/mL) | 8.689 |
| R square | 0.9962 |

### Example 2: Preparation of anti-human CD70 alpaca VHH antibody

### 1. Immune alpaca

2 mg/mL CD70-alIgG1Fc fusion protein as an antigen was mixed and emulsified with an equal volume of complete Freund's adjuvant (Sigma-Aldrich), and adult alpacas were subcutaneously immunized with 500 µg of antigen per animal. After the initial immunization, a booster immunization was performed every 20 days, and a total of four subcutaneous immunizations were performed. Seven days after the fourth immunization, 100 mL of whole blood was collected intravenously, and PBMCs were isolated.

### 2. Detection of serum titer:

Before each booster immunization, 10 mL of blood was collected from the vein, and the cells were removed by centrifugation, and the serum was retained. 50 ng/well of CD70-his protein (ACRO Biosystems) was added to the ELISA microwell plate, and coated overnight at 4°C. The above mentioned microwell plate was washed three times with PBS, 1% BSA/PBS was added, 200 µL/well, the above mixture was blocked at 37°C for 1 hour. The serially diluted alpaca serum was added and bound for 1 hour at 37°C. The above mixture was washed three times with PBST, 100 µL of 1:5000 diluted HRP-goat anti-alpaca IgG1-Fc (Jackson ImmunoResearch) was added, and bound for 1 hour at 37°C. The above mixture was washed with PBST three times, 100 µL/well TMB chromogen solution was added for development at 37°C for 10 minutes, 100 µL/well ELISA stop solution was added, and the absorbance value at OD₄₅₀ was obtained by a microplate reader. The serum titer detection results are shown by Table 2

**Table 2.Detection of serum titer after immunization of alpaca**

| Dilution multiple | negative | secondary immunizatio n | third immunizatio n | forth immunizatio n |
|---|---|---|---|---|
| 1:2K | 0.043 | 1.2551 | 1.411 | 2.1077 |
| 1:4K | 0.0423 | 0.6624 | 0.9337 | 1.2117 |
| 1:8K | 0.0418 | 0.391 | 0.5955 | 0.8136 |
| 1:16K | 0.0429 | 0.2568 | 0.276 | 0.3979 |
| 1:32K | 0.0444 | 0.1311 | 0.152 | 0.1965 |
| 1:64K | 0.0443 | 0.1056 | 0.1077 | 0.1505 |
| 1:128K | 0.0439 | 0.0733 | 0.0728 | 0.0858 |
| blank | 0.0461 | 0.0442 | 0.0434 | 0.0508 |

### 3. Construction of immune library

### 3.1 Alpaca PBMC total cDNA acquisition

Total RNA from alpaca PBMC was extracted using Trizol RNA extraction kitUsing the RNA as a template, the first-strand cDNA was synthesized using the SuperScript^{™} IV First-Strand Synthesis System Kit.3.2 VHH gene amplificationUsing the cDNA as a template, the heavy chain gene was amplified by PCR using the upstream primers of the heavy chain variable domain and the downstream constant region CH2 primers (VH-F, CH2-R). In a 50 µL reaction system, 25 µL PrimeSTAR MAX master mix (Takara), 2.5 µL upstream primer (25 pmol), 2.5 µL downstream primer (25 pmol), 1.5 µL DMSO, 0.5 µL cDNA and 18 µL ddH₂O were added respectively. The PCR reaction was performed according to the following program: 98°C pre-denaturation for 1 minute, followed by temperature cycling, 98°C denaturation for 110 seconds, 60°C annealing for 15 seconds, 72°C extension for 30 minutes, 25 cycles, and 72°C final extension for 10 minutes.

The amplified VHH-CH2 gene was recovered using a DNA gel recovery kit, and the electrophoresis results are shown by Figure 3. 100ng VHH-CH2 was taken as a template, and the VHH gene was amplified by PCR via using the upstream primer VH-F and downstream primer VH-R. In a 50 µL reaction system, 25 µL PrimeSTAR MAX master mix (Takara), 2.5 µL upstream primer (25 pmol), 2.5 µL downstream primer (25 pmol), 1.5 µL DMSO, 0.5 µL VH-CH2 DNA and 18 µL ddH₂O were added respectively. The PCR reaction was performed according to the following program: 98°C pre-denaturation for 1 minute, followed by temperature cycling, 98°C denaturation for 110 seconds, 60°C annealing for 15 seconds, 72°C extension for 30 minutes, 25 cycles, and 72°C final extension for 10 minutes. The amplified VHH gene fragment was recovered using the gel recovery kit, and the electrophoresis results are shown by Figure 4.

### 3.3 Construction of immune library

The VHH gene fragment and the pcomb3X-TT vector (Scripps Research Institute, USA) were digested with SfiI DNA endonuclease, respectively. In a 50 µL reaction system, 2 µL of SfiI, 5 µL of 10xbuffer, 3 µg of DNA were added, and ddH₂O was added to 50 µL. After mixing well, the above reaction system was incubated at 50°C for 3 hours.

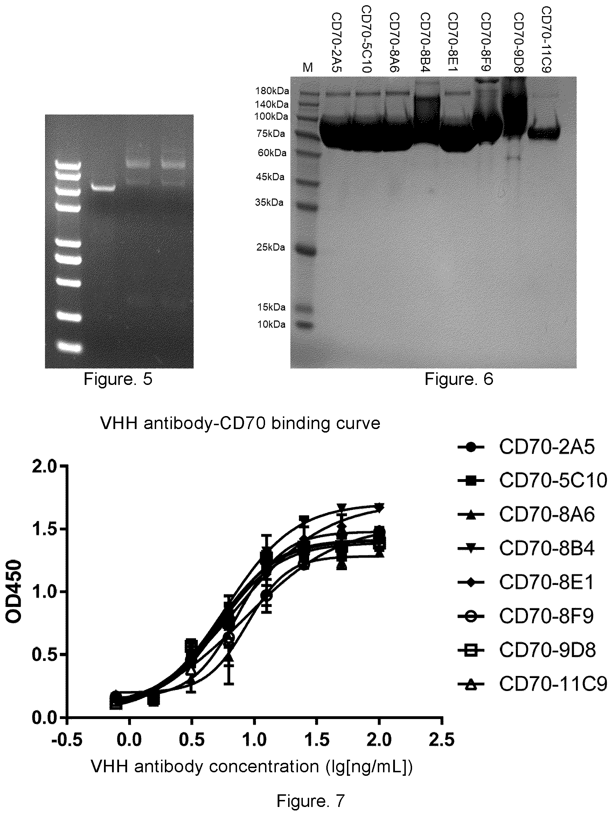

The digested VHH gene fragment and pcomb3X vector were recovered by using the DNA gel recovery kit. The digested VHH gene fragment and pcomb3X vector were cyclized by using T4 ligase. In a 50 µL reaction system, 1 µL of T4 ligase, 5 µL of 10×buffer, 150 ng of VHH gene, 1000 ng of pComb3X vector were added, and ddH₂O was added to 50 µL. After mixing well, the above misture was incubated at 4°C for 16 hours. A small amount of product was taken to verify the ligation efficiency by agarose gel electrophoresis, and the electrophoresis results are shown by Figure 5.

10 µL of the above ligated cyclization product was added into the self-made TG1 electrotransformation competent cell, and then using an electroporator for electroporation transformation. 10 µL of electrotransformed bacteria were taken out, diluted appropriately and streaked on a plate containing ampicillin to count and calculate the size of the phage antibody library. The remaining electrotransformed bacteria were added to 2×YT medium containing 100 µg/mL ampicillin and 2% glucose, and cultured in a heating incubator. After the cultivation, the above substances were centrifuged at 4000 G for 10 minutes at 4°C, an appropriate amount of glycerol was added to the precipitated bacteria and the precipitated bacteria was stored at -80°C as the antibody strain library. The scFv immune library with a storage capacity of more than 9E+9 was accumulated through multiple electroporations.

### 4. Screening CD70 Antibody

### 4.1 Recombinant human CD70 protein coupled to streptavidin magnetic beads

The avi-tag of the recombinant human CD70 protein was biotin-modified with a biotinylation kit (Yijin Biology) according to the kit instructions to obtain biotinylated CD70 protein. 10 µg of the above biotin-modified recombinant protein was added to 100 µL streptavidin magnetic beads (DynaBeads 280) washed three times with PBS, placed on a rotary shaker at a speed of 18 rpm, and coupled at room temperature for 30 minutes, then washed 3 times with PBS.

### 4.2 Blocking the phage library and magnetic beads.

0.5mL 1% BSA/PBS was added to 0.5mL phage library, placed on a rotary shaker at a speed of 18 rpm, and rotated and blocked at room temperature for 1 hour. These phages are Input1. At the same time, 100 µL of uncoupled protein DynaBeads 280 was taken, washed with PBS for 3 times, 1 mL of 1% BSA/PBS was added, and incubated with rotation for 1 hour according to the above conditions. In addition, 1 mL of 1% BSA/PBS was added to the above CD70-coupled magnetic beads and rotated to block for 1 hour under the above conditions.

### 4.3 Negative panning

Negative panning is necessary to remove antibodies that interact with the beads. The BSA-blocked phage library was mixed with uncoupled antigen magnetic beads, and the above mixture was rotated and incubated for 1 hour according to the above conditions. After the incubation, the phage magnetic bead mixture was placed on the magnetic stand, and after the magnetic beads were all attached to the wall, the supernatant was transferred to a new EP tube.

### 4.4 Positive panning

The above-mentioned blocked magnetic beads coupled with CD70 protein was added to the phage supernatant after negative panning for positive panning, and the above mixture was incubated with rotation at room temperature for 1 hour according to the above conditions. After the incubation, the magnetic beads were washed with 1 mL of PBST (0.1% Tween-20in PBS), and the washing was repeated 10 times. After washing, 1 mL of 100 mM glycine (pH 2.0) was added, then the above magnetic beads placed on a rotary shaker, set the speed to 18 rpm, and rotated for elution for 10 minutes. After the elution, the EP tube was placed on the magnetic stand, and transfer the eluate to a new EP tube after the magnetic beads are all attached to the wall. 0.2 mL of 1M Tris-HCl solution (pH 8.0) was added to the eluate for neutralization. The neutralized eluate was added to 30 mL TG1 bacterial solution with an OD₆₀₀ of about 0.6 and settled for infection for 30 minutes, then the M13KO7 phage, which is 20 times the number of bacterial cells, was added and settled for infection for 30 minutes, and finally 100 mL 2YT culture medium and ampicillin and kanamycin with a final concentration of 100 µg/mL were added and the above mixture was cultured overnight at 30 °C and 220 rpm. The next day, the phage were harvested according to the above-mentioned method for harvesting the phage library, and the phage obtained at this time is Input2.

### 4.5 Repeat positive panning

The above panning method was repeated twice, that is, Input2 was subjected to the next round of negative panning and positive panning to obtain Input3. The difference is that after infecting TG1 with the eluate obtained from panning on Input3, M13KO7 was not added, but 10 µL of the bacterial solution was taken for gradient dilution, and 100 µL of the three dilution gradients of 103, 104, and 105 were used to coat 2YT/amp plate, cultivated overnight at 30 °C; the rest of the bacterial solution was cultivated at 30 °C, 220 rpm overnight.

4.6 Screening positive antibodies by ELISA.TG1 monoclonal antibodies from the above plates were randomly selected using a toothpick and transfered to a deep well plate containing 600 µ L 2YT/amp. The deep well plate was covered with a breathable membrane and incubated at 37 °C and 220 rpm for 3 hours. Then, the breathable membrane was removed and IPTG with a final concentration of 1mM was added to the well, and the well palte was incubated overnight at 30 °C and 220rpm. The ELISA plate was coated with recombinant human CD70 protein at a concentration of 100 ng per well. The next day, the deep-well plate was centrifuged at 4000rpm for 10 minutes to remove the culture medium in the well to retain the precipitated bacteria, 100 µL of TES solution (20% sucrose, 0.1 mM EDTA, 50 mM Tris-HCl, pH 8.0) was added to each well, the well palte was shaked to resuspend the bacteria cells, and then ice-bath for 30 minutes, 200 µL of ultrapure water was added, and the bacteria cells were shaked and mixed, centrifuged at 4000 rpm for 10 minutes. At this time, the supernatant solution in the deep well plate is the periplasmic cavity extract containing the antibody. The ELISA plate was washed three times with a plate washer, then 200 µL of 1% BSA/PBS was added, and the ELISA plate was blocked at 37°C for 1 hour. The blocking solution in the ELISA plate was removed, 100 µL of the above periplasmic cavity extract was added, then the ELISA plate was incubated at 37°C for 1 hour, and washed 3 times with a plate washer, HRP-conjugated-GoatantiHA (horseradish peroxidase-labeled goat anti-HA antibody) solution was added, then the ELISA plate was incubated at 37°C for 1 hour, washed 3 times with a plate washer, 100 µL TMB color developing solution was added for development at 37°C for 10 minutes, and 100 µL 1M hydrochloric acid was added to stop. The absorbance value at OD₄₅₀ was obtained with a microplate reader, and performing Sanger sequencing on the clones whose reading value is 3 times higher than the background value to obtain the gene sequence of the antibody.

### 4.7 Verification of positive clones

According to the sequencing results, clones with large differences in the amino acid sequence of the antibody CDR3 was selected to re-inoculate and induce overnight, and the above-mentioned ELISA method was used to verify again whether the selected clones could bind to CD70. Finally, eight VHH antibody sequences of CD70-11C9, CD70-8E1, CD70-8F9, CD70-9D8, CD70-2A5, CD70-5C10, CD70-8A6, and CD70-8B4 were obtained.

The amino acid sequence of the heavy chain variable region of CD70-2A5 is shown by SEQ ID NO:21.

The amino acid sequence of the heavy chain variable region of CD70-5C10 is shown by SEQ ID NO:22.

The amino acid sequence of the heavy chain variable region of CD70-8A6 is shown by SEQ ID NO:23.

The amino acid sequence of the heavy chain variable region of CD70-8B4 is shown by SEQ ID NO:24.

The amino acid sequence of the heavy chain variable region of CD70-8E1 is shown by SEQ ID NO:25.

The amino acid sequence of the heavy chain variable region of CD70-8F9 is shown by SEQ ID NO:26.

The amino acid sequence of the heavy chain variable region of CD70-9D8 is shown by SEQ ID NO:27.

The amino acid sequence of the heavy chain variable region of CD70-11C9 is shown by SEQ ID NO:28.

### Example 3: Expression of recombinant VHH antibodies

Eight VHH antibody genes, CD70-2A5, CD70-5C10, CD70-8A6, CD70-8B4, CD70-8E1, CD70-8F9, CD70-9D8, and CD70-11C9, were constructed into pcDNA3.1-huIgG1-Fc by homologous recombination, to obtain recombinant VHH-huIgG1-Fc. 293F cells were transiently transfected with the above vectors for eukaryotic expression, and the supernatant was harvested and purified with HiTrap Protein A HP/AKTA pure100. The purified protein was concentrated with an ultrafiltration tube and the buffer was replaced with PBS, and the protein purity was detected by SDS-PAGE electrophoresis, the electrophoresis results are shown by Figure 6.

Example 4: Determination of the binding curves of recombinant human CD70 protein and 8 VHH antibodies. The specific operation of the ELISA experiment is as follows: 100 ng/well of the recombinant human CD70 protein prepared above was added to the microtiter plate, and coated overnight at 4°C. Washed three times with PBS, 1% BSA/PBS was added, 200 µL/well, then the above microtiter plate was blocked at 37°C for 1 hour. After washing the plate with 100 µL of PBS, the above four scFv proteins were added in gradient dilution, and combined at 37 °C for 1 hour. Washed three times with PBST, 100 µL of 1:5000 diluted HRP-goat anti-human IgG (Fab specific) was added, and bound for 1 hour at 37 °C. Washed with PBST three times, 100 µL/well TMB chromogen solution was added for development at 37°C for 10 minutes, 100 µL/well ELISA stop solution was added, the absorbance value at OD₄₅₀ was obtained with microplate reader, the results are shown by Figure 7 and Table 3.

**Table 3 test results**

| | CD70-2A5 | CD70-5C10 | CD70-8A6 | CD70-8B4 | CD70-8E1 | CD70-8F9 | CD70-9D8 | CD70-11C9 |
|---|---|---|---|---|---|---|---|---|
| EC50(n g/mL) | 5.548 | 5.293 | 8.728 | 5.954 | 7.054 | 8.482 | 4.972 | 7.377 |
| R square | 0.986 | 0.9869 | 0.9936 | 0.9929 | 0.9657 | 0.991 | 0.9807 | 0.9596 |

### Example 5: Affinity Determination

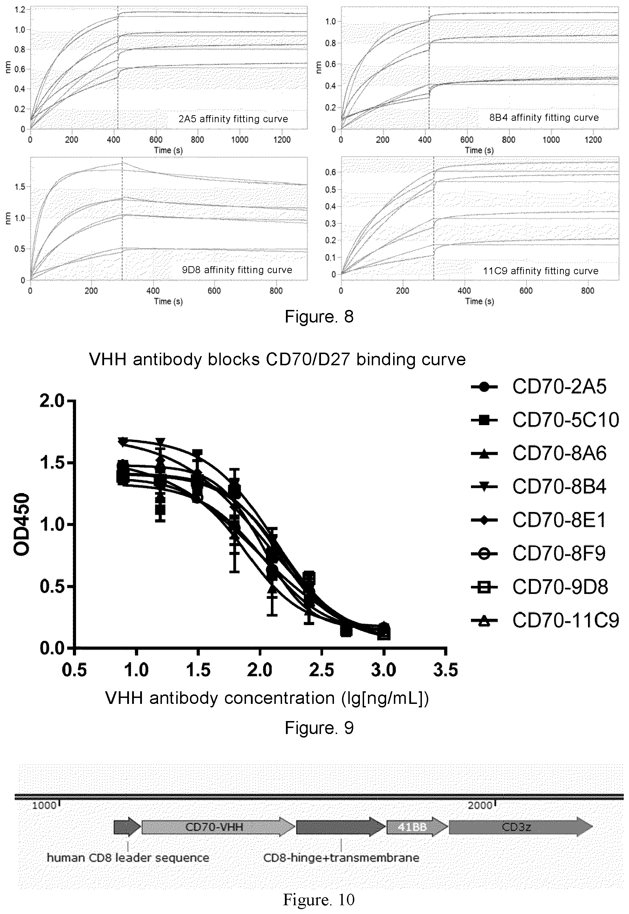

The affinity of four VHH-huFc antibodies CD70-2A5, CD70-8B4, CD70-9D8, and CD70-11C9 to human CD70 was analyzed by Octet K2 molecular interaction analyzer. The SA probe was immobilized with 200 µL 100 nM biotinylated recombinant human CD70 protein at a height of 1 nM. Four purified VHH-huFc antibodies were used as analytes, and four concentrations of 200nM, 100nM, 50nM, and 25nM were set for affinity determination. The results are shown by Figure 8 and Table 4.

**Table 4, VHH-huFc affinity determination results**

| Name | KD (M) | Kon (1/Ms) | Koff (1/s) |
|---|---|---|---|
| CD70-2A5 | <1E-12 | 3.67E+4 | <1.0E-7 |
| CD70-8B4 | <1E-12 | 4.55E+4 | <1.0E-7 |
| CD70-9D8 | 1.83E-9 | 1.29E+5 | 2.35E-4 |
| CD70-11C9 | <1E-12 | 2.64E+5 | <1.0E-7 |

### Example 6: VHH-huFc blocks CD27/CD70 binding

The specific operation of the ELISA experiment is as follows: 100 ng/well of the recombinant human CD70 protein was added to the microtiter plate, and coated overnight at 4°C. Washed three times with PBS, 1% BSA/PBS was add, 200 µL/well, the above microtiter plate was blocked at 37°C for 1 hour. 100 ng of biotinylated CD70 recombinant protein was incubated with 1 µg, 500 ng, 250 ng, and 125 ng of the above-mentioned VHH-huFc antibody for 1 hour, respectively. After washing the plate with 200 µL PBS, the above-mentioned CD70/VHH-Fc mixture was added, and bound at 37 °C for 1 hour. The above plate was washed three times with PBST, 100 µL 1:200 diluted SA-HRP was added and bound for 1 hour at 37°C. Washed with PBST three times, 100 µL/well TMB chromogen solution was added for development at 37°C for 10 minutes, 100 µL/well ELISA stop solution was added, the absorbance value at OD₄₅₀ was obtained with microplate reader, the results are shown by Figure 9 and Table 5.

### Example 7: Preparation of retrovirus stock solution containing anti-human CD70 chimeric antigen receptor element.

### 1. Preparation of chimeric antigen receptor targeting human CD70 antigen

Gene synthesis or cloning of chimeric antigen receptor sequences containing anti human CD70 antigen single-chain antibody scFv, hinge region, transmembrane region and intracellular signal segment. Its structure is shown by Figure 10. According to the difference of loaded VHH, chimeric antigen receptors were named as CD70-2A5-BBz, CD70-5C10-BBz, CD70-8A6-BBz, CD70-8B4-BBz, CD70-8E1-BBz, CD70-8F9-BBz, CD70-9D8-BBz and CD70-11C9-BBz, the amino acid sequences of which are shown by SEQ ID NO:29-36 respectively, and the nucleotide sequences are shown by SEQ ID NO:42-49 respectively. At the same time, the scFv of the known CD70 antibody (clone number: ARGX110) reported in the reference was selected to construct a chimeric antigen receptor as a control, named ARGX-BBz, and its nucleic acid sequence is shown by SEQ ID NO:52.

Using the reverse transcription vector MSGV as a backbone vector, a retroviral plasmids expressing the chimeric antigen receptor of CD70-2A5-BBz, CD70-5C10-BBz, CD70-8A6-BBz, CD70-8B4-BBz, CD70-8E1-BBz, CD70-8F9-BBz, CD70- 9D8-BBz and CD70-11C9-BBz and ARGX-BBz clone was constructed. The clones with correct sequencing were selected, the bacterial solution was inoculated into 300 mL LB medium, shaked overnight, and the plasmid extraction was completed according to the instructions of the NucleoBond Xtra Maxi EF kit.

### 2.Retroviral packaging.

Retrovirus was packaged with cationic polymer PEI (Polyplus) as follows: PEI and retroviral packaging plasmid (viral master plasmid, Gag-pol, 10A1) was diluted with serum-free DMEM respectively; and then PEI/DMEM was added to the plasmid/DMEM mixture, vortex to mix, and the above mixture was placed at room temperature for 15 minutes; the plasmid-PEI complex was added to the pre-plated 293T cells. The medium was changed 16 hours after transfection, and the virus supernatant was collected after 48 hours, filtered through a 0.45 um filter, aliquoted into 15 mL centrifuge tubes, and stored at -80°C for later use.

### Example 8: Preparation of CD70 CAR-T cells and determination of CAR positive rate

### 1. PBMC isolation and activation.

The peripheral blood of volunteers was collected, and PBMCs were obtained by separating with Ficoll separation medium, and the cell density was adjusted to 1x106/mL with X-VIVO (LONZA) medium containing 5% AB serum. Pre-coat the TC-treated 6-well plate with 1 mL of coating solution containing 50 ng/mL anti-human CD3 antibody (Beijing Tongli Haiyuan) and 50 ng/mL CD28 antibody (Beijing Tongli Haiyuan) at 37°C for 2h, and the coating solution was removed before use. Cells were inoculated into a 6-well plate coated with antibodies at 1 mL/well, and 100 IU/mL IL2 (Beijing Shuanglu) was added to stimulate culture for 48 hours before virus infection.

### 2. Virus stock solution infection and culture.

The activated T cells were adjusted to 5×10⁵/mL, 1 mL T cells and 1 mL virus stock solution were added respectively to the 24-well plate, 1µL polybrene was added to each well, the 24-well plate was centrifuged at 32 °C, 2500 rpm for 1.5h. The supernatant was discarded, and 1 mL of T cell medium (containing IL-2 100 IU/mL) was added to each well. The culture plate was placed in a 37°C, 5% CO2 incubator. 24 hours after infection, transfered to a 6-well plate, the cell density was observed every day, and T cell culture medium containing IL-2 100 IU/mL was added in time to maintain the density of T cells at about 1×10⁶/mL to expand the cells.

### 3. CAR positive rate detection.

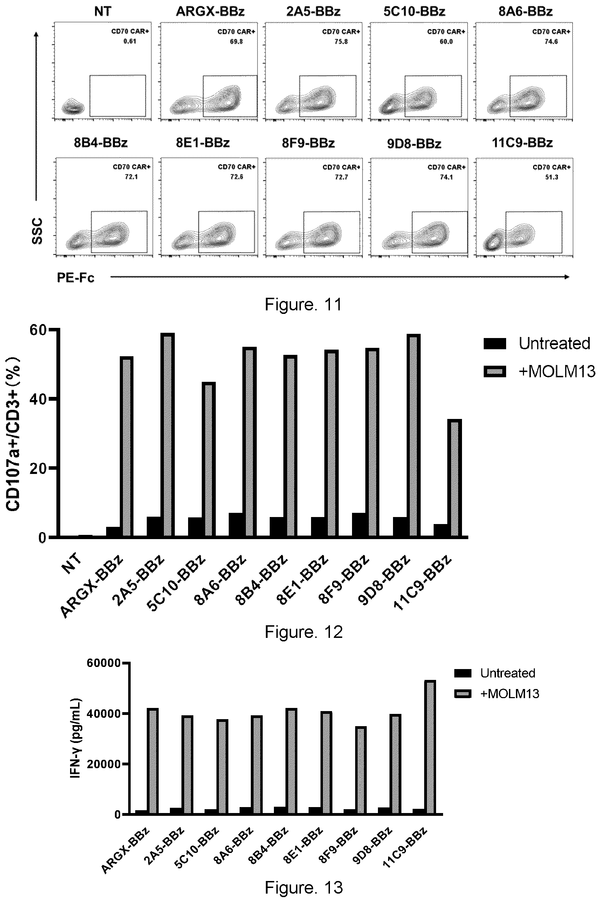

The CAR positive rate of the retrovirus-infected T lymphocytes were detected 72 hours after virus infection. For the chimeric antigen receptor group containing CD70-2A5-BBz, CD70-5C10-BBz, CD70-8A6-BBz, CD70-8B4-BBz, CD70-8E1-BBz, CD70-8F9-BBz, CD70-9D8-BBz, CD70-11C9-BBz and ARGX-BBz clone and the negative uninfected control group NT, 1×10⁶ cells from the above two groups respectively, were taken and centrifuged to remove the medium, the cells were washed once with PBS, and resuspended in 100 µL of the flow loading tube (BD). Fc-labeled CD70 antigen (1:100) was added and incubated for 30 minutes at 4 °C. After washing the cells once with PBS, the secondary antibody PE-Fc was added according to the recommended ratio and incubated for 30 minutes in the dark. After the cells were washed once with PBS, the cells were resuspended in 200 µL of PBS and tested on the machine. The results of flow cytometric analysis of CAR-T positive rate are shown by Figure 11.

### Example 9: Functional analysis of CAR-T cells based on anti-human CD70.

### 1. Analysis of CD107a expression of anti-human CD70 CAR-T cells

After co-incubating CAR-T cells containing different antibody clones and NT cells with target cells (CD70-positive cell line MOLM13) at an effect-to-target ratio of 1:1 (3×10⁵ effector cells and target cells), the expression of CD107a was detected by flow cytometry to evaluate the degranulation response of CAR-T cells after being stimulated by target cells. After the effector cells and target cells were mixed and incubated in a 37°C, 5% CO2 incubator for 4 hours, the proportions of cells expressing CD107a to the number of CD3+ cells in each group of samples were detected by flow cytometry. The results of flow cytometric analysis of CD107a expression are shown by Figure 12.

### 2. Detection of cytokine secretion ability of anti-human CD70 CAR-T cells.

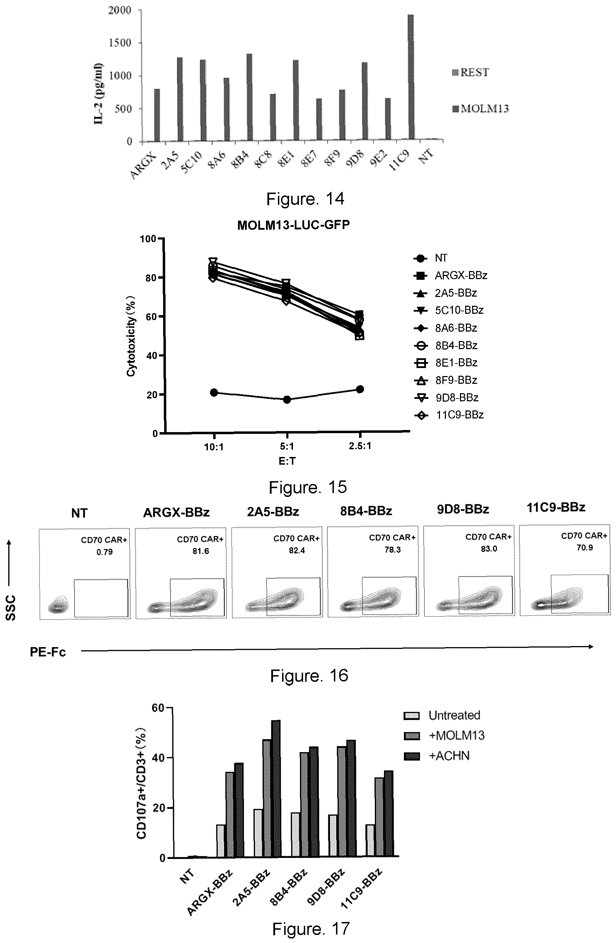

After incubating CAR-T cells containing different antibody clones with target cells (CD70-positive cell line MOLM13) at an effect-to-target ratio of 1:1 (both effector cells and target cells were 1×105) for 24 hours, the supernatant was collected and the secretion of IFN-γ and IL-2 were detected using ELISA (enzyme-linked immunosorbent assay) method. IFN-γ was detected using BD IFN-γ and IL-2 kits, and the experimental steps were performed according to the product instructions. The detection results of IFN-γ secretion are shown by Figure 13. The detection results of IL-2 are shown by Figure 14.

### 3. Anti-human CD70 CAR-T cytotoxicity experiment.

The CAR-T killing toxicity test evaluates the in vitro function of CAR-T cells by detecting the killing effect of CAR-T cells on target cells in vitro. With different effector-target ratios (based on 3x104 target cells, the effector-target ratios were 10:1, 5:1 and 2.5:1), respectively, T cells were co-cultured with CD70-positive target cells stably expressing firefly luciferase MOLM13-LUC-GFP, and a negative control group (NT) in which target cells were mixed with untransfected CAR element T cells was set at the same time. After overnight incubation, the luciferase reaction substrate was added to the culture system, the fluorescence value was detected, and the killing efficiency was calculated by the following formula: killing efficiency=(1-fluorescence value of experimental well/fluorescence value of control well)×100%. The experimental grouping and analysis results are shown by Figure 15.

### Example 10: Drug efficacy test of anti-human CD70 CAR-T cell injection in liquid.

According to the method described in Example 8 of the present description, CAR-T cells of four clones of CD70-2A5/8B4/9D8/11C9 were prepared for drug efficacy tests in animals. And detecting the CAR-T positivity rate and in vitro killing efficiency of the prepared CAR-T cells against ACHN cells (human renal cell carcinoma cell line, expressing CD70). Meanwhile, MOLM13 cells were used as a control, as follows:

### 1. Detection of CAR positive rate.

The CAR positive rate of the retrovirus-infected T lymphocytes was detected 72 hours after virus infection. For the chimeric antigen receptor group containing CD70-2A5-BBz, CD70-8B4-BBz, CD70-9D8-BBz, CD70-11C9-BBz and ARGX-BBz clones and the negative uninfected control NT, 1×10⁶ cells from the above two groups respectively, were taken and centrifuged to remove the medium, washed once with PBS, and resuspended in 100 µL in a flow loading tube (BD). Fc-labeled CD70 antigen (1:100) was added and incubated for 30 minutes at 4 °C. After washing the cells once with PBS, the secondary antibody PE-Fc was added according to the recommended ratio and incubated for 30 minutes in the dark. After the cells were washed once with PBS, the cells were resuspended in 200 µL of PBS and tested on the machine. The results of flow cytometric analysis of CAR-T positive rate are shown by Figure 16.

### 2.1. Analysis of CD107a expression of anti-human CD70 CAR-T cells

After co-incubating CAR-T cells containing different antibody clones and NT cells with target cells (CD70-positive cell line MOLM13) at an effect-to-target ratio of 1:1 (3×10⁵ effector cells and target cells), the expression of CD107a was detected by flow cytometry to evaluate the degranulation response of CAR-T cells after being stimulated by target cells. After the effector cells and target cells were mixed and incubated in a 37°C, 5% CO2 incubator for 4 hours, the proportions of cells expressing CD107a to the number of CD3+ cells in each group of samples were detected by flow cytometry. The results of flow cytometric analysis of CD107a expression are shown by Figure.17.

### 3. Anti-human CD70 CAR-T cell killing experiments on ACHN and MOLM13 cells.

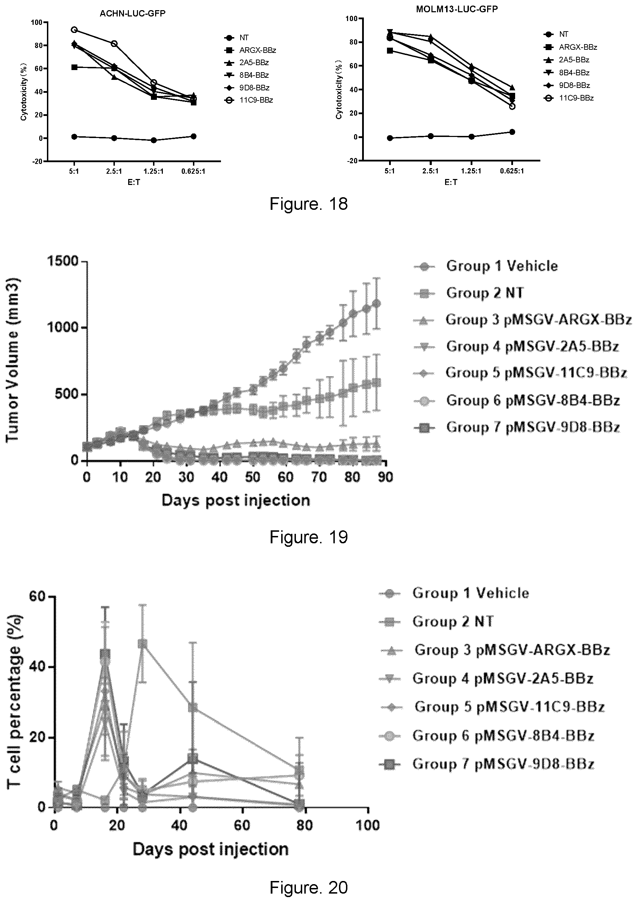

CAR-T cells containing CD70-2A5, CD70-8B4, CD70-9D8, CD70-11C9 and NT cells were treated with different effect-target ratios (based on 3x104 target cells, the effect-target ratios were 10:1, 5: 1 and 2.5:1) and co-cultured with CD70-positive ACHN and MOLM13 target cells (ACHN-LUC-GFP, MOLM13-LUC-GFP) stably expressing firefly luciferase, respectively. After overnight incubation, the luciferase reaction substrate was added to the culture system, the fluorescence value was detected, and the killing efficiency was calculated by the following formula: killing efficiency=(1-fluorescence value of experimental well/fluorescence value of control well)×100%. The experimental grouping and analysis results are shown by Figure 18.

### 4. Drug efficacy experiment in mice

For the experiment, 48 female mice bearing NOG tumors (subcutaneously inoculated with ACHN cells of human kidney cancer cell line) were used. When the tumor volume reaches about 100 mm³, they were randomly divided into 6 groups according to the tumor size, and intravenously administered NT cell injection and CD70 CAR-T cell injection of 5 different clones, code-named P376, 2A5, 11C9, 8B4, 9D8. Each group was administered 200 µL per mouse, and the dose was 3×10⁷ total cells per mouse. Clinical observation was performed once a day during the test period; about 100 µL of anticoagulant blood was taken from the orbits of mice in each group on 1D, 7D, 16D, and 22D for flow cytometry detection to detect the retention of T cells in the mice; tumor volume was measured and the living conditions of the mice were recorded about twice a week. (When the tumor volume of the mice reached 2000 mm³, they needed to be euthanized in time.) The test was observed until D24, the tumor volume monitoring results are shown by Figure 19, and the T cell percentage detection results are shown by Figure 20.

The above results show that the CD70-targeted CAR-T cells constructed based on the new VHH antibody specifically binding to CD70 provided by the present description have strong immune function. Compared with the control CAR-T (clone number ARGX110) cells, the CAR-T cells have better CD107a expression, IFN-γ and IL-2 secretion and specific killing function on target cells, the drug effect in vivo is remarkable.

The above descriptions are only preferred embodiments of the present description, and are not intended to limit the present description. For those skilled in the art, the present description may have various modifications and changes. Any modifications, equivalent replacements, improvements, etc. made within the spirit and principles of the present description shall be included within the protection scope of the present description.

## Claims

1. A CD70 binding molecule, comprising an anti-CD70 nanoantibody or an antigen-binding fragment thereof, wherein the complementarity-determining region (CDRs) of the anti-CD70 nanoantibody comprise CDR1, CDR2 and CDR3, wherein CDR1 comprises the sequence shown by any one of SEQ ID NO: 1-7, CDR2 comprises the sequence shown by any one of SEQ ID NO: 8-13, and CDR3 comprises the sequence shown by any one of SEQ ID NO: 14-20,
preferably, the CD70 binding molecule also comprises one or more features selected from the group consisting of:
the heavy chain variable region sequence of the anti-CD70 nanoantibody is as shown by any one of SEQ ID NO: 21-28,
the CD70 binding molecules are monovalent or multivalent nanoantibodies or single-domain antibodies, or multispecific nanoantibodies or single-domain antibodies comprising one, two or more of the anti-CD70 nanoantibodies or antigen-binding fragment thereof,
the nanoantibody is a camelid heavy chain antibody or a shark heavy chain antibody,
the nanoantibody further comprises a heavy chain constant region,
the CD70 binding molecule is a chimeric or fully human antibody.

2. A chimeric antigen receptor comprising an optional signal peptide sequence, the CD70 binding molecule according to claim 1, a hinge region, a transmembrane region and an intracellular region,
preferably, the intracellular region comprises an intracellular costimulatory domain and/or an intracellular signaling domain,
preferably, from the N-terminus to the C-terminus, the chimeric antigen receptor comprises a signal peptide, the CD70 binding molecule according to claim 1, a hinge region, a transmembrane region, an intracellular costimulatory domain and an intracellular signaling domain.

3. A nucleic acid molecule comprising a sequence selected from any of the group consisting of:
(1) the coding sequence of the CD70 binding molecule according to claim 1 or the chimeric antigen receptor according to claim 2;
(2) a complementary sequence of (1);
(3) a 5-50 bp fragment of any sequence of (1) or (2),
preferably, the fragment is a primer.

4. A nucleic acid construct, wherein the nucleic acid construct comprises the nucleic acid molecule according to claim 3,
preferably, the nucleic acid construct is a cloning vector, an expression vector or an integration vector.

5. A host cell, wherein the host cell:
(1) expresses and/or secrets the CD70 binding molecule according to claim 1 or the chimeric antigen receptor according to claim 2;
(2) comprises nucleic acid molecule according to claim 3; and/or
(3) comprises the nucleic acid construct according to claim 4,
preferably, the host cell is an immune effector cell, more preferably a T cell.

6. A method of producing the CD70 binding molecule according to claim 1 or the chimeric antigen receptor according to claim 2, comprising:
culturing the host cell according to claim 5 under conditions suitable for producing the CD70 binding molecule, and
optionally purifying the CD70 binding molecule or chimeric antigen receptor from the culture.

7. A pharmaceutical composition comprising the CD70 binding molecule according to claim 1, the chimeric antigen receptor according to claim 2, the nucleic acid molecule according to claim 3, the nucleic acid construct according to claim 4, or the host cell according to claim 5, and a pharmaceutically acceptable excipient.

8. Use of the CD70 binding molecule according to claim 1, the chimeric antigen receptor according to claim 2, the nucleic acid molecule according to claim 3, the nucleic acid construct according to claim 4, or the host cell according to claim 5 in the preparation of activated immune cells, or in the manufacture of a medicament for the prevention or treatment of a disease or condition associated with CD70 expression,
preferably, the disease or condition is selected from one or more of the following: renal cell carcinoma, acute myelogenous leukemia, non-Hodgkin's lymphoma, multiple myeloma, mantle cell lymphoma, diffuse large cell lymphoma, follicular lymphoma, pancreatic cancer, breast cancer, glioblastoma.

9. A kit for detecting CD70, the kit comprising the CD70 binding molecule according to claim 1, the nucleic acid molecule according to claim 3, the nucleic acid construct according to claim 4 or the host cell according to claim 5,
preferably, the kit further comprises a reagent for detecting the binding of CD70 to the CD70 binding molecule; more preferably, the reagent for detecting the binding is a detectable label that can be linked to the CD70 binding molecule.

10. Use of the CD70 binding molecule according to claim 1 in the preparation of a kit for detecting CD70 in samples, evaluating the therapeutic effect of a medicament, or diagnosing a cancer.
